# EUROPÄISCHE PATENTSCHRIFT

(11) **EP 3 266 770 B1**
(45) Veröffentlichungstag und Bekanntmachung des Hinweises auf die Patenterteilung: **13.05.2020**
(21) Anmeldenummer: 17178013.3
(22) Anmeldetag: 27.06.2017
(51) Int. Cl.: C07D 295/037, C07D 401/04, C07D 401/12, C07D 401/14

(54) **VERFAHREN ZUR HERSTELLUNG EINER N-METHYLSUBSTITUIERTEN TRIACETONAMINVERBINDUNG**
METHOD FOR PRODUCING AN N-METHYL SUBSTITUTED TRIACETONE AMINE COMPOUND
PROCEDE DE FABRICATION D'UNE LIAISON DE TRIACETONAMINE N-METHYL-SUBSTITUE

(30) Priorität: 07.07.2016 DE 102016212379
(43) Veröffentlichungstag der Anmeldung: 10.01.2018
(73) Patentinhaber: Evonik Operations GmbH, 45128 Essen (DE)
(72) Erfinder: Minke, Katharina, 45130 Essen (DE); Willy, Benjamin, 40211 Düsseldorf (DE); Nissen, Felix, 48301 Nottuln (DE); Neumann, Manfred, 45770 Marl (DE)
(74) Vertreter: Evonik Patent Association

(56) Entgegenhaltungen:
- EP-A1- 0 729 947
- EP-A1- 3 048 097
- EP-A2- 0 302 020

## Beschreibung

Die vorliegende Erfindung beschreibt ein Verfahren zur Herstellung einer *N*-methylsubstituierten Triacetonaminverbindung (Triacetonamin = "TAA"). Die vorliegende Erfindung beschreibt dabei insbesondere ein Verfahren zur Methylierung des im Ring befindlichen Stickstoffes, wie beispielsweise in der Reaktionsgleichung **<1>** dargestellt (R ist dabei zum Beispiel ein Alkylrest, ein Alkoxyrest, ein Aminrest oder auch eine OH-Gruppe):

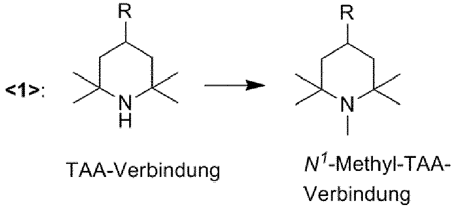

### Hintergrund der Erfindung

Methylierte Derivate des 2,2,6,6-Tetramethylpiperidins sind besonders in der Anwendung als "*hindered amine light stabilizers*" von besonderer Bedeutung. Durch die Methylierung wird eine Anwendung auch unter sauren Bedingungen ermöglicht. Kommerzielle Produkte, welche über *N*-methylierte 2,2,6,6-Tetramethylpiperidin-Gruppen verfügen, sind z. B. Tinuvin® 292 [eine Mischung aus 1-(Methyl)-8-(1,2,2,6,6-pentamethyl-4-piperidinyl)-sebacat und *Bis*-(1,2,2,6,6-pentamethyl-4-piperidinyl)-sebacat] oder Cyasorb® UV-3529 (CAS NUMBER 193098-40-7).

Im Stand der Technik sind verschiedene Verfahren zur Methylierung von Aminen beschrieben.

So beschreiben z. B. Kopka, I. E., et al., J. Org. Chem. 1980, 45, 4616 - 4622 und Minatti, A., et al., J. Org. Chem. 2007, 72, 9253 - 9258 die Umsetzung von Aminen mit Methylhalogeniden. Diese ist schematisch in der Reaktionsgleichung **<2>** dargestellt (R ist dabei ein Rest, wie er in Bezug auf die Reaktionsgleichung **<1>** definiert ist):

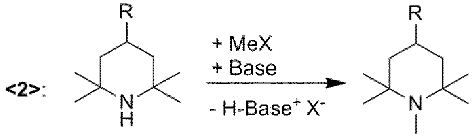

Nachteilig an dem in Reaktionsgleichung **<2>** dargestellten Verfahren ist dabei, dass zur Freisetzung des Produktes neben dem Methylhalogenid mindestens ein Äquivalent einer geeigneten Base eingesetzt werden muss. Dies führt zudem zur Bildung der entsprechenden Salze, welche dann als Abfallprodukt entstehen. Problematisch ist zudem, dass eine selektive Alkylierung zum tertiären Amin im Allgemeinen nicht möglich ist, da auch eine Überalkylierung zum entsprechenden quartären Ammoniumsalz stattfinden kann.

Ein weiteres im Stand der Technik beschriebenes Verfahren ist die Eschweiler-Clarke-Reaktion (z. B. Lutz, W. B., et al., J. Org. Chem. 1962, 27, 1695 - 1703; EP 0 729 947 A1; WO 2004/072035 A1; WO 2005/123679 A2). Bei diesem Verfahren wird das Amin in Gegenwart von Ameisensäure mit Formaldehyd umgesetzt. Die Ameisensäure fungiert dabei als Reduktionsmittel und wird in CO₂ überführt. Damit die Reaktion abläuft, wird zudem im Allgemeinen ein Äquivalent Base benötigt. Die Reaktion ist schematisch in der folgenden Reaktionsgleichung **<3>** wiedergegeben (R ist dabei ein Rest, wie er in Bezug auf die Reaktionsgleichung **<1>** definiert ist):

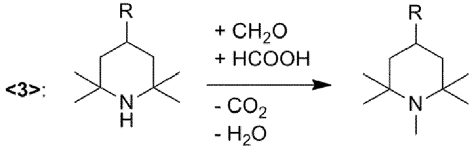

Nachteilig ist hier die nötige Verwendung von Ameisensäure. Zudem führt die Verwendung der Base wiederum zur Generierung eines entsprechenden Abfallstromes.

Eine weitere im Stand der Technik (z. B. WO 2004/089913 A1, WO 2008/101979 A1) beschriebene Möglichkeit zur *N*-Methylierung stellt die Umsetzung mit Formaldehyd in Anwesenheit von Borhydriden dar (z. B. Natriumborhydrid). Die Reaktion ist schematisch in der folgenden Reaktionsgleichung **<4>** wiedergegeben (R ist dabei ein Rest, wie er in Bezug auf die Reaktionsgleichung **<1>** definiert ist):

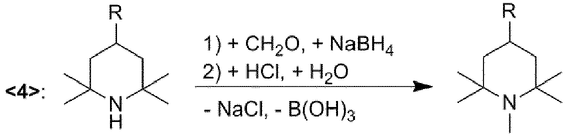

Nachteilig ist hier die nötige Verwendung der Borhydride. Bei der Aufarbeitung entstehen große Mengen an Borsäure oder Borsäurederivaten als Abfallstrom.

Die Aufgabe der vorliegenden Erfindung bestand deshalb darin, ein Verfahren zur Herstellung einer *N*-methylsubstituierten Triacetonaminverbindung zur Verfügung stellen, welches die vorstehend genannten Nachteile nicht aufweist.

Es wurde nun überraschend ein Verfahren gefunden, welches die vorbeschriebene Aufgabe löst.

### Beschreibung der Erfindung

Die vorliegende Erfindung betrifft in einem ersten Aspekt ein Verfahren gemäß den folgenden Punkten 1.1 bis 1.11.
1.1 Verfahren zur Herstellung einer *N*-methylsubstituierten Triacetonaminverbindung,
   dadurch gekennzeichnet, dass man mindestens eine Triacetonaminverbindung **(I)** mit Formaldehyd unter reduktiven Bedingungen umsetzt,
   wobei die Triacetonaminverbindung **(I)** ausgewählt ist aus der Gruppe bestehend aus den chemischen Strukturen **(I-A)**, **(I-B)**, **(I-C)**, **(I-D)**, **(I-E)**, **(I-F)**, **(I-G)**, **(I-H)** mit 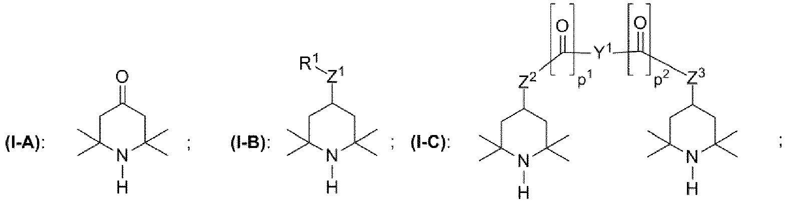 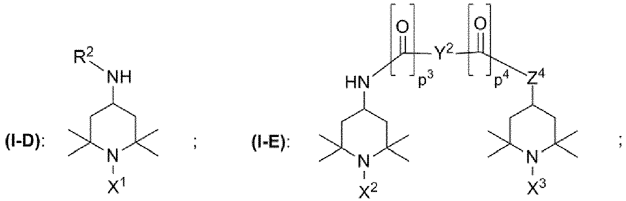 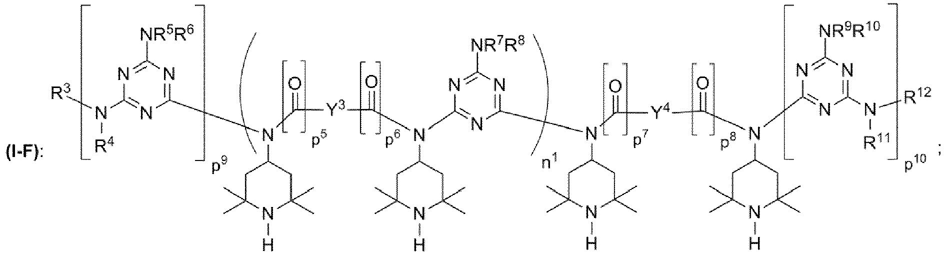 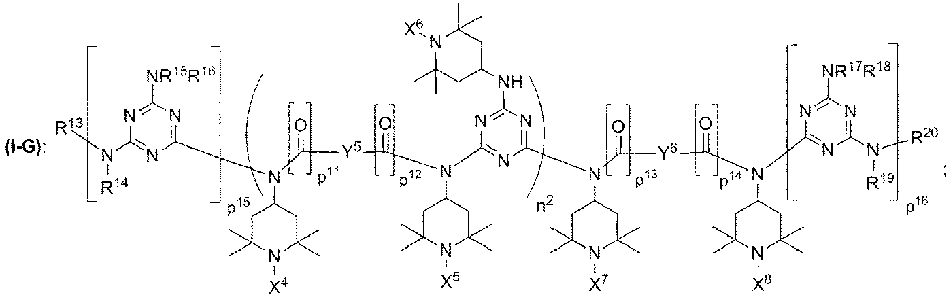 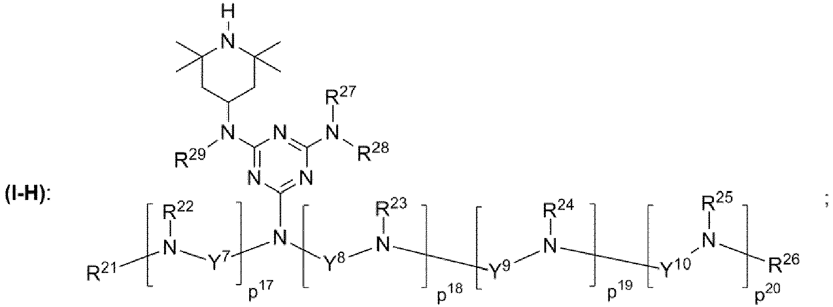
   wobei n¹, n² unabhängig voneinander jeweils eine ganze Zahl aus dem Bereich 1 bis 20 ist;
   wobei p¹, p², p³, p⁴, p⁵, p⁶, p⁷, p⁸, p⁹, p¹⁰, p¹¹, p¹², p¹³, p¹⁴, p¹⁵, p¹⁶, p¹⁷, p¹⁸, p¹⁹, p²⁰ unabhängig voneinander jeweils 0 oder 1 ist;
   wobei X¹ ausgewählt ist aus der Gruppe bestehend aus OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei X², X³ ausgewählt sind aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen, und wobei X², X³ jeweils unabhängig voneinander ausgewählt sind, bis auf die Ausnahme, dass nicht gilt: X² = X³ = Wasserstoff;
   wobei X⁴, X⁵, X⁶, X⁷, X⁸ unabhängig voneinander jeweils ausgewählt sind aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y¹, Y², Y³, Y⁴, Y⁵, Y⁶, Y⁷, Y⁸, Y⁹, Y¹⁰ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen
         mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i)**, **(ii)** mit 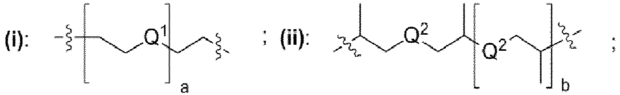 aufweist, wobei
         Q1, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y¹ auch eine direkte Bindung sein kann, falls mindestens einer von p¹ und p² den Wert 1 aufweist,
   und wobei Y² auch eine direkte Bindung sein kann, falls mindestens einer von p³ und p⁴ den Wert 1 aufweist,
   und wobei Y³ auch eine direkte Bindung sein kann, falls mindestens einer von p⁵ und p⁶ den Wert 1 aufweist,
   und wobei Y⁴ auch eine direkte Bindung sein kann, falls mindestens einer von p⁷ und p⁸ den Wert 1 aufweist,
   und wobei Y⁵ auch eine direkte Bindung sein kann, falls mindestens einer von p¹¹ und p¹² den Wert 1 aufweist,
   und wobei Y⁶ auch eine direkte Bindung sein kann, falls mindestens einer von p¹³ und p¹⁴ den Wert 1 aufweist;
   und wobei Z¹, Z², Z³, Z⁴ unabhängig voneinander jeweils ausgewählt sind aus der Gruppe bestehend aus -O-, -S-, -NR³⁰-;
   wobei die Reste R¹, R², R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ unabhängig voneinander jeweils ausgewählt sind aus der Gruppe bestehend aus
      Wasserstoff, unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii)**, **(iv)**, **(v)**, **(vi)**, **(vii)**, **(viii)**, **(ix)** mit 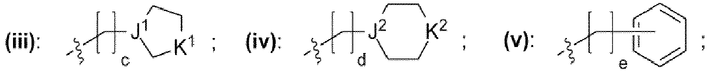 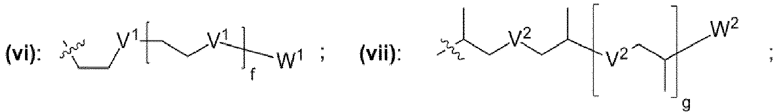 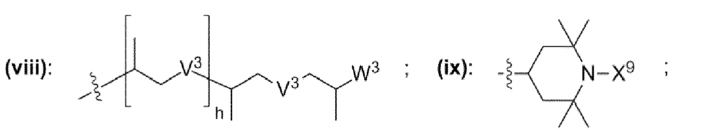 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   wobei die Reste R⁷, R⁸ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur **(x)** sein kann,
   und wobei, wenn p¹⁰ = 1, -NR¹¹R¹² auch ein Rest der chemischen Struktur **(x)** sein kann,
   und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur **(x)** wie folgt definiert ist: 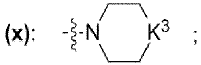 wobei K³ ausgewählt ist aus der Gruppe bestehend aus -O-, -S-, -NH-, -N(CH₃)-, -N(CH₂CH₃)-, wobei K³ bevorzugt -O- ist;
   wobei die Reste R²¹, R²², R²³, R²⁴, R²⁵, R²⁶ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen,
      einer Gruppe mit der chemischen Struktur **(xi)** mit 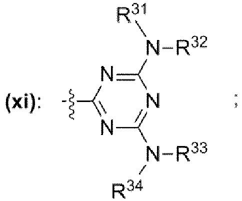
   wobei die Reste R²⁷, R²⁸, R²⁹, R³¹, R³², R³³, R³⁴ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(xii)**, **(xiii)**, **(xiv)**, **(xv)**, **(xvi)**, **(xvii)**, **(xviii)** mit 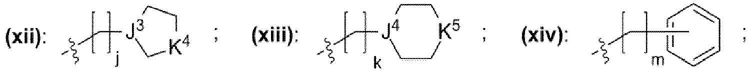 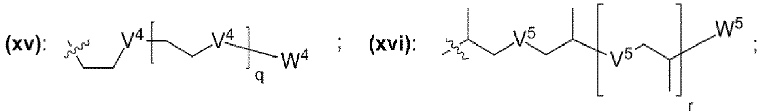 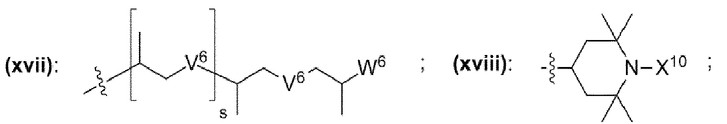 aufweist,
         wobei J³, J⁴ unabhängig voneinander jeweils ausgewählt aus der Gruppe bestehend aus CH, N sind,
         wobei K⁴, K⁵ unabhängig voneinander jeweils ausgewählt aus der Gruppe bestehend aus -O-, -NH-, -N(CH₃)-, -N(CH₂CH₃)-, -S-, -CH₂- sind,
         wobei V⁴, V⁵, V⁶ unabhängig voneinander jeweils ausgewählt aus der Gruppe bestehend aus -O-, -S-, -NH-, -NR'"- mit R'" = unverzweigte oder verzweigte Alkylgruppe mit 1 bis 6 Kohlenstoffatomen sind,
         wobei W⁴, W⁵, W⁶ unabhängig voneinander jeweils ausgewählt aus der Gruppe bestehend aus H, Methyl, Ethyl sind,
         wobei j, k, m, q, r, s unabhängig voneinander jeweils eine ganze Zahl aus dem Bereich 0 bis 50 ist,
         wobei X¹⁰ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
         wobei in den chemischen Strukturen **(xii)**, **(xiii)**, **(xiv)**, **(xv)**, **(xvi)**, **(xvii)**, **(xviii)** mindestens ein an ein Kohlenstoffatom gebundener Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 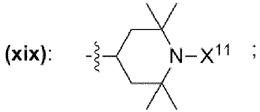 sein können, wobei X¹¹ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
1.2 Verfahren nach Punkt 1.1, wobei p¹ = p² = p³ = p⁴ = p⁵ = p⁶ = p⁷ = p⁸ = p¹¹ = p¹² = p¹³ = p¹⁴ = 0 ist und wobei p⁹, p¹⁰, p¹⁵, p¹⁶, p¹⁷, p¹⁸, p¹⁹, p²⁰ unabhängig voneinander jeweils 0 oder 1 ist.
1.3 Verfahren nach Punkt 1.1 oder 1.2, wobei
   Y¹, Y², Y³, Y⁴, Y⁵, Y⁶, Y⁷, Y⁸, Y⁹, Y¹⁰ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche
      mindestens einen gesättigten Ring aus 3 bis 30 Kohlenstoffatomen aufweist.
1.4 Verfahren nach einem oder mehreren der Punkte 1.1 bis 1.3, wobei die Reste R¹, R², R³, R⁴ R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 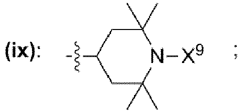 aufweist,
   wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei die Reste R⁷, R⁸ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur **(x)** sein kann,
   und wobei, wenn p¹⁰ = 1, -NR¹¹R¹² auch ein Rest der chemischen Struktur **(x)** sein kann,
   und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur **(x)** wie folgt definiert ist: 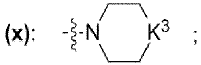 wobei K³ ausgewählt ist aus der Gruppe bestehend aus -O-, -S-, -NH-, -N(CH₃)-, -N(CH₂CH₃)-, wobei K³ bevorzugt -O- ist;
   wobei die Reste R²¹, R²², R²³, R²⁴, R²⁵, R²⁶ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen,
      einer Gruppe mit der chemischen Struktur **(xi)** mit 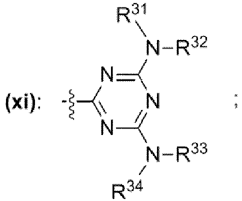
   wobei die Reste R²⁷, R²⁸, R²⁹, R³¹, R³², R³³, R³⁴ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur **(xviii)** mit 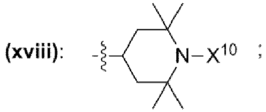 aufweist,
         wobei X¹⁰ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 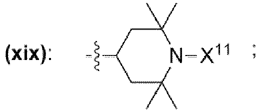 sein können, wobei X¹¹ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
1.5 Verfahren nach einem oder mehreren der Punkte 1.1 bis 1.4, wobei
   X⁴ = X⁵ = X⁶ = X⁷ = X⁸ = X⁹ = X¹⁰ = X¹¹ = Wasserstoff ist.
1.6 Verfahren nach einem oder mehreren der Punkte 1.1 bis 1.5, wobei die Triacetonaminverbindung **(I)** ausgewählt ist aus der Gruppe bestehend aus den chemischen Strukturen **(I-A)**, **(I-B)**, **(I-C)**, **(I-D)**, **(I-E)**.
1.7 Verfahren nach einem oder mehreren der Punkte 1.1 bis 1.6, wobei die Triacetonaminverbindung **(I)** ausgewählt ist aus der Gruppe bestehend aus den chemischen Strukturen **(I-A)**, **(I-B)**, **(I-D)**,
   und wobei Z¹ ausgewählt ist aus der Gruppe bestehend aus -O-, -S-, -NR³⁰-;
   wobei die Reste R¹, R², R³⁰ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
1.8 Verfahren nach einem oder mehreren der Punkte 1.1 bis 1.7, wobei die Triacetonaminverbindung **(I)** ausgewählt ist aus der Gruppe bestehend aus den chemischen Strukturen **(I-A)**, **(I-B)**, **(I-D)**
   und wobei Z¹ ausgewählt ist aus der Gruppe bestehend aus -O-, -NR³⁰-;
   wobei die Reste R¹, R², R³⁰ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen, bevorzugt 1 bis 6 Kohlenstoffatomen.
1.9 Verfahren nach einem oder mehreren der Punkte 1.1 bis 1.8, wobei Formaldehyd als Gas, als wässrige Lösung oder als Feststoff eingesetzt wird, bevorzugt als wässrige Lösung oder als Feststoff, noch bevorzugter als wässrige Lösung.
1.10 Verfahren nach einem oder mehreren der Punkte 1.1 bis 1.9, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
1.11 Verfahren nach einem oder mehreren der Punkte 1.1 bis 1.10, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem zweiten Aspekt ein Verfahren gemäß den folgenden Punkten 2.1 bis 2.4.
2.1 Verfahren zur Herstellung einer *N*-methylsubstituierten Triacetonaminverbindung, dadurch gekennzeichnet, dass man eine Triacetonaminverbindung **(I)** mit Formaldehyd unter reduktiven Bedingungen umsetzt,
   wobei die Triacetonaminverbindung **(I)** die chemische Struktur **(I-A)** mit 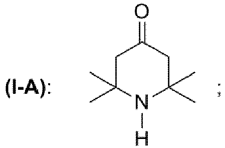 aufweist, und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
2.2 Verfahren nach Punkt 2.1, wobei Formaldehyd als Gas, als wässrige Lösung oder als Feststoff eingesetzt wird, bevorzugt als wässrige Lösung oder als Feststoff, noch bevorzugter als wässrige Lösung.
2.3 Verfahren nach einem oder mehreren der Punkte 2.1 bis 2.2, wobei man die Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
2.4 Verfahren nach einem oder mehreren der Punkte 2.1 bis 2.3, wobei man die Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem dritten Aspekt ein Verfahren gemäß den folgenden Punkten 3.1 bis 3.8.
3.1 Verfahren zur Herstellung einer *N*-methylsubstituierten Triacetonaminverbindung,
   dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Struktur **(I-B)** mit 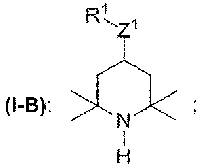 aufweist,
   wobei Z¹ ausgewählt ist aus der Gruppe bestehend aus -O-, -S-, -NR³⁰-;
   wobei die Reste R¹, R³⁰ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, - N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(C H₃)(CH₂CH₃) ersetzt sein kann,
      einem Rest, der eine chemischen Struktur ausgewählt aus der Gruppe bestehend aus **(iii)**, **(iv)**, **(v)**, **(vi)**, **(vii)**, **(viii)**, **(ix)** mit 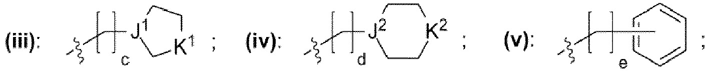 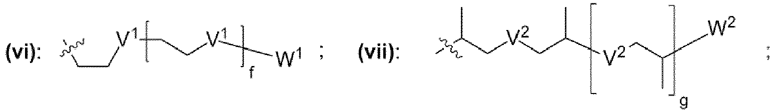 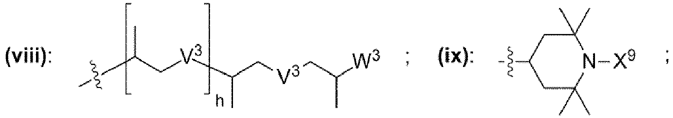 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann, und
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
3.2 Verfahren nach Punkt 3.1, wobei die Reste R¹, R³⁰ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 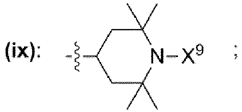 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
3.3 Verfahren nach Punkt 3.1 oder 3.2, wobei X⁹ = Wasserstoff ist.
3.4 Verfahren nach einem oder mehreren der Punkte 3.1 bis 3.3,
   wobei die Reste R¹, R³⁰ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
3.5 Verfahren nach einem oder mehreren der Punkte 3.1 bis 3.4, wobei
   Z¹ ausgewählt ist aus der Gruppe bestehend aus -O-, -NR³⁰-;
   wobei die Reste R¹, R³⁰ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen.
3.6 Verfahren nach einem oder mehreren der Punkte 3.1 bis 3.5, wobei Formaldehyd als Gas, als wässrige Lösung oder als Feststoff eingesetzt wird, bevorzugt als wässrige Lösung oder als Feststoff, noch bevorzugter als wässrige Lösung.
3.7 Verfahren nach einem oder mehreren der Punkte 3.1 bis 3.6, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
3.8 Verfahren nach einem oder mehreren der Punkte 3.1 bis 3.7, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem vierten Aspekt ein Verfahren gemäß den folgenden Punkten 4.1 bis 4.10.
4.1 Verfahren zur Herstellung einer *N*-methylsubstituierten Triacetonaminverbindung,
   dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Struktur **(I-C)** mit 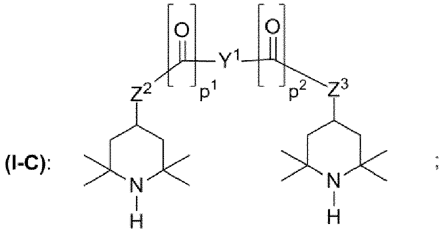 aufweist,
   wobei p¹, p² unabhängig voneinander jeweils 0 oder 1 ist;
   wobei Y¹ ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i)**, **(ii)** mit 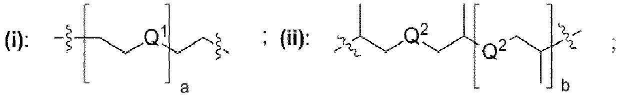 aufweist, wobei
         Q1, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y¹ auch eine direkte Bindung sein kann, falls mindestens einer von p¹ und p² den Wert 1 aufweist,
   und wobei Z², Z³ unabhängig voneinander jeweils ausgewählt sind aus der Gruppe bestehend aus -O-, -S-, -NR³⁰-;
   wobei der Rest R³⁰ ausgewählt ist aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii)**, **(iv)**, **(v)**, **(vi)**, **(vii)**, **(viii)**, **(ix)** mit 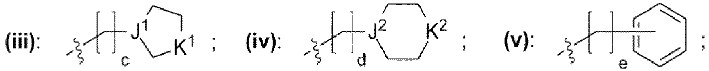 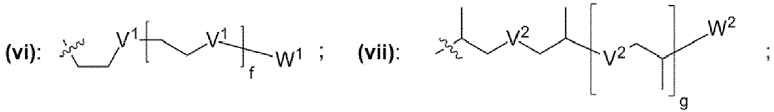 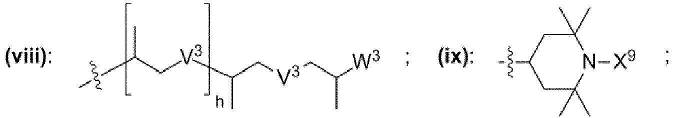 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
         -OH, -NH₂, -OCH₃, -OCH₂CH₃, -NH(CH₃), -N(CH₃)₂, -NH(CH₂CH₃), -N(CH₂CH₃)₂, -N(CH₃) (CH₂CH₃) ersetzt sein kann,
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
4.2 Verfahren nach Punkt 4.1, wobei p¹ = p² = 0 ist.
4.3 Verfahren nach Punkt 4.1 oder 4.2, wobei
   Y¹ ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
4.4 Verfahren nach einem oder mehreren der Punkte 4.1 bis 4.3, wobei der Rest R³⁰ ausgewählt ist aus der Gruppe bestehend aus
   Wasserstoff, unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃) ersetzt sein kann,
   einem Rest, der eine chemischen Struktur **(ix)** mit 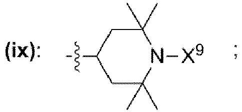 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
4.5 Verfahren nach einem oder mehreren der Punkte 4.1 bis 4.4, wobei X⁹ = Wasserstoff ist.
4.6 Verfahren nach einem oder mehreren der Punkte 4.1 bis 4.5, wobei der Rest R³⁰ ausgewählt ist aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
4.7 Verfahren nach einem oder mehreren der Punkte 4.1 bis 4.6, wobei der Rest R³⁰ ausgewählt ist aus der Gruppe bestehend aus
   Wasserstoff,
   unverzweigte oder verzweigte Alkylgruppe mit 1 bis 12, bevorzugt 1 bis 6, Kohlenstoffatomen.
4.8 Verfahren nach einem oder mehreren der Punkte 4.1 bis 4.7, wobei Formaldehyd als Gas, als wässrige Lösung oder als Feststoff eingesetzt wird, bevorzugt als wässrige Lösung oder als Feststoff, noch bevorzugter als wässrige Lösung.
4.9 Verfahren nach einem oder mehreren der Punkte 4.1 bis 4.8, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
4.10 Verfahren nach einem oder mehreren der Punkte 4.1 bis 4.9, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem fünften Aspekt ein Verfahren gemäß den folgenden Punkten 5.1 bis 5.8.
5.1 Verfahren zur Herstellung einer *N*-methylsubstituierten Triacetonaminverbindung,
   dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Strukturen **(I-D)** mit 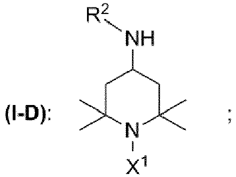
   wobei X¹ ausgewählt ist aus der Gruppe bestehend aus OH, -O· , unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei der Rest R² ausgewählt ist aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(C H₃)(CH₂CH₃) ersetzt sein kann,
      einem Rest, der eine chemischen Struktur ausgewählt aus der Gruppe bestehend aus **(iii)**, **(iv)**, **(v)**, **(vi)**, **(vii)**, **(viii)**, **(ix)** mit 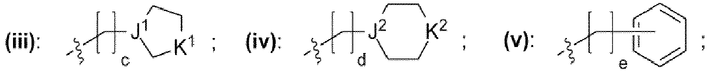 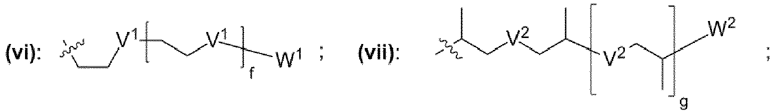 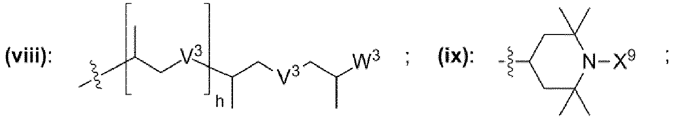 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
         -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
5.2 Verfahren nach Punkt 5.1, wobei der Rest R² ausgewählt ist aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃) ersetzt sein kann,
   einem Rest, der eine chemischen Struktur **(ix)** mit 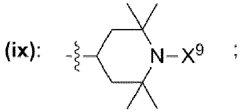 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
5.3 Verfahren nach Punkt 5.1 oder 5.2, wobei X⁹ = Wasserstoff ist.
5.4 Verfahren nach einem oder mehreren der Punkte 5.1 bis 5.3, wobei der Rest R² ausgewählt ist aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
5.5 Verfahren nach einem oder mehreren der Punkte 5.1 bis 5.4, wobei der Rest R² ausgewählt ist aus der Gruppe bestehend aus
   Wasserstoff,
   unverzweigte oder verzweigte Alkylgruppe mit 1 bis 12, bevorzugt 1 bis 6, Kohlenstoffatomen.
5.6 Verfahren nach einem oder mehreren der Punkte 5.1 bis 5.5, wobei Formaldehyd als Gas, als wässrige Lösung oder als Feststoff eingesetzt wird, bevorzugt als wässrige Lösung oder als Feststoff, noch bevorzugter als wässrige Lösung.
5.7 Verfahren nach einem oder mehreren der Punkte 5.1 bis 5.6, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
5.8 Verfahren nach einem oder mehreren der Punkte 5.1 bis 5.7, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem sechsten Aspekt ein Verfahren gemäß den folgenden Punkten 6.1 bis 6.10.
6.1 Verfahren zur Herstellung einer *N*-methylsubstituierten Triacetonaminverbindung, dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Strukturen **(I-E)** mit 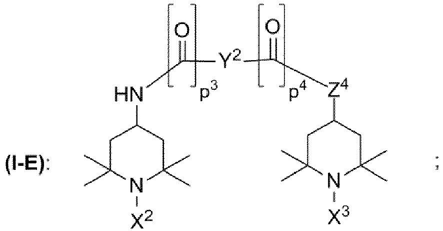 aufweist,
   wobei p³, p⁴ unabhängig voneinander jeweils 0 oder 1 ist;
   wobei X², X³ ausgewählt sind aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen, und wobei X², X³ jeweils unabhängig voneinander ausgewählt sind, bis auf die Ausnahme, dass nicht gilt: X² = X³ = Wasserstoff;
   wobei Y² ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i)**, **(ii)** mit 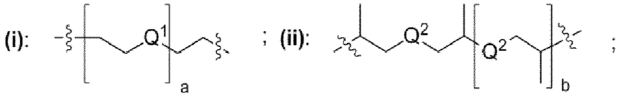 aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y² auch eine direkte Bindung sein kann, falls mindestens einer von p³ und p⁴ den Wert 1 aufweist,
   und wobei Z⁴ ausgewählt ist aus der Gruppe bestehend aus -O-, -S-, -NR³⁰-;
   wobei der Rest R³⁰ ausgewählt ist aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii)**, **(iv)**, **(v)**, **(vi)**, **(vii)**, **(viii)**, **(ix)** mit 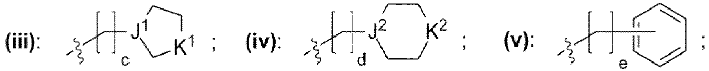 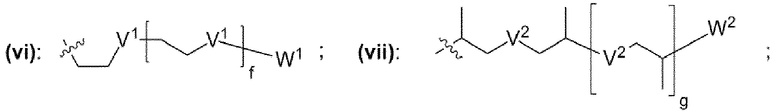 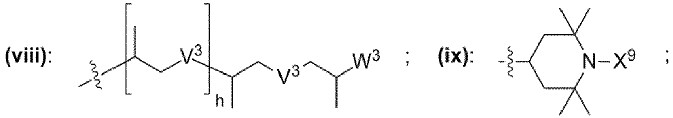 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
         -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
6.2 Verfahren nach Punkt 6.1, wobei p³ = p⁴ = 0 ist.
6.3 Verfahren nach Punkt 6.1 oder 6.2, wobei
   Y² ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
6.4 Verfahren nach einem oder mehreren der Punkte 6.1 bis 6.3, wobei der Rest R³⁰ ausgewählt ist aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃) ersetzt sein kann,
   einem Rest, der eine chemischen Struktur **(ix)** mit 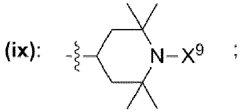 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
6.5 Verfahren nach einem oder mehreren der Punkte 6.1 bis 6.4, wobei der Rest R³⁰ ausgewählt ist aus der Gruppe bestehend aus
   Wasserstoff,
   unverzweigte oder verzweigte Alkylgruppe mit 1 bis 12 Kohlenstoffatomen,
   einem Rest, der eine chemischen Struktur **(ix)** mit 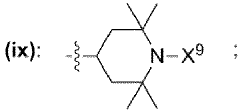 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
6.6 Verfahren nach einem oder mehreren der Punkte 6.1 bis 6.5, wobei der Rest R³⁰ ausgewählt ist aus der Gruppe bestehend aus Wasserstoff, unverzweigte oder verzweigte Alkylgruppe mit 1 bis 6 Kohlenstoffatomen.
6.7 Verfahren nach einem oder mehreren der Punkte 6.1 bis 6.6, wobei X⁹ = Wasserstoff ist.
6.8 Verfahren nach einem oder mehreren der Punkte 6.1 bis 6.7, wobei Formaldehyd als Gas, als wässrige Lösung oder als Feststoff eingesetzt wird, bevorzugt als wässrige Lösung oder als Feststoff, noch bevorzugter als wässrige Lösung.
6.9 Verfahren nach einem oder mehreren der Punkte 6.1 bis 6.8, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
6.10 Verfahren nach einem oder mehreren der Punkte 6.1 bis 6.9, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem siebten Aspekt ein Verfahren gemäß den folgenden Punkten 7.1 bis 7.9.
7.1 Verfahren zur Herstellung einer *N*-methylsubstituierten Triacetonaminverbindung,
   dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Struktur **(I-F)** mit 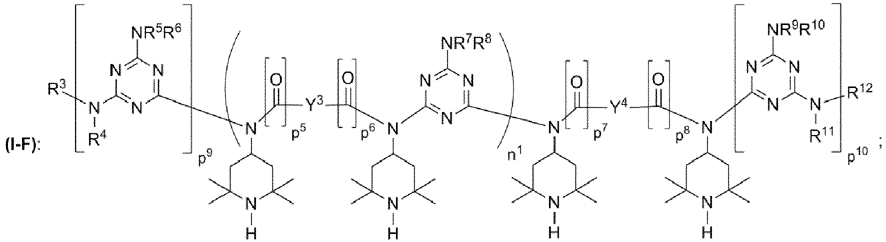
   wobei n¹ eine ganze Zahl aus dem Bereich 1 bis 20 ist;
   wobei p⁵, p⁶, p⁷, p⁸, p⁹, p¹⁰ unabhängig voneinander jeweils 0 oder 1 ist;
   wobei Y³, Y⁴ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen
         mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe
         bestehend aus **(i)**, **(ii)** mit 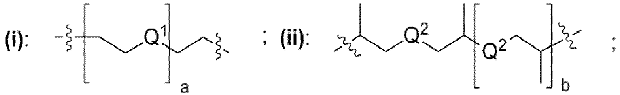 aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y³ auch eine direkte Bindung sein kann, falls mindestens einer von p⁵ und p⁶ den Wert 1 aufweist,
   und wobei Y⁴ auch eine direkte Bindung sein kann, falls mindestens einer von p⁷ und p⁸ den Wert 1 aufweist,
   wobei die Reste R³, R⁴, R⁵, R⁶, R⁹, R¹⁰, R¹¹, R¹² unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii)**, **(iv)**, **(v)**, **(vi)**, **(vii)**, **(viii)**, **(ix)** mit 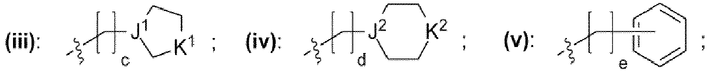 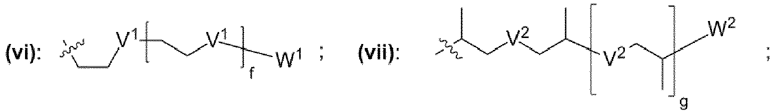 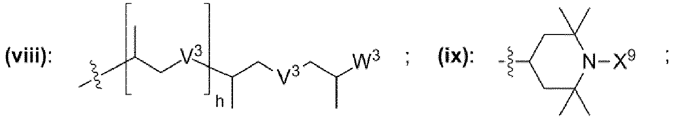 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
         -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   wobei die Reste R⁷, R⁸ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur **(x)** sein kann,
   und wobei, wenn p¹⁰ = 1, -NR¹¹R¹² auch ein Rest der chemischen Struktur **(x)** sein kann,
   und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur **(x)** wie folgt definiert ist: 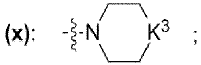 wobei K³ ausgewählt ist aus der Gruppe bestehend aus -O-, -S-, -NH-, -N(CH₃)-, -N(CH₂CH₃)-, wobei K³ bevorzugt -O- ist,
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
7.2 Verfahren nach Punkt 7.1, wobei p⁵ = p⁶ = p⁷ = p⁸ = 0 ist und wobei p⁹, p¹⁰ unabhängig voneinander jeweils 0 oder 1, ist; bevorzugt ist dabei p⁵ = p⁶ = p⁷ = p⁸ = 0 ist und mindestens einer von p⁹, p¹⁰ ist 1 (wobei logischerweise für den Fall, in dem nur einer vor p⁹, p¹⁰ = 1 ist, der andere von p⁹, p¹⁰ = 0 ist); noch bevorzugter ist p⁵ = p⁶ = p⁷ = p⁸ = 0 und p⁹ = p¹⁰ = 1.
7.3 Verfahren nach Punkt 7.1 oder 7.2, wobei Y³, Y⁴ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
7.4 Verfahren nach einem oder mehreren der Punkte 7.1 bis 7.3, wobei die Reste R³, R⁴, R⁵, R⁶, R⁹, R¹⁰, R¹¹, R¹² unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 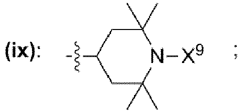 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei die Reste R⁷, R⁸ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur **(x)** sein kann,und wobei, wenn p¹⁰ = 1, -NR¹¹R¹² auch ein Rest der chemischen Struktur **(x)** sein kann, und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur **(x)** wie folgt definiert ist: 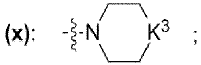 wobei K³ ausgewählt ist aus der Gruppe bestehend aus -O-, -S-, -NH-, -N(CH₃)-, -N(CH₂CH₃)-, wobei K³ bevorzugt -O- ist.
7.5 Verfahren nach einem oder mehreren der Punkte 7.1 bis 7.4, wobei die Reste R³, R⁴, R⁵, R⁶, R⁹, R¹⁰, R¹¹, R¹² unabhängig voneinander jeweils ausgewählt sind aus der Gruppe bestehend aus
   Wasserstoff,
   unverzweigte oder verzweigte Alkylgruppe mit 1 bis 12 Kohlenstoffatomen,
   einem Rest, der eine chemischen Struktur **(ix)** mit 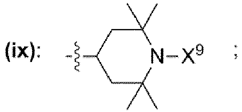 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei die Reste R⁷, R⁸ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   und wobei der Rest -NR⁷R⁸ auch ein Rest der chemischen Struktur **(x)** sein kann mit 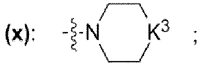 wobei K³ = -O-.
7.6 Verfahren nach einem oder mehreren der Punkte 7.1 bis 7.5, wobei X⁹ = Wasserstoff ist.
7.7 Verfahren nach einem oder mehreren der Punkte 7.1 bis 7.6, wobei Formaldehyd als Gas, als wässrige Lösung oder als Feststoff eingesetzt wird, bevorzugt als wässrige Lösung oder als Feststoff, noch bevorzugter als wässrige Lösung.
7.8 Verfahren nach einem oder mehreren der Punkte 7.1 bis 7.7, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
7.9 Verfahren nach einem oder mehreren der Punkte 7.1 bis 7.8, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem achten Aspekt ein Verfahren gemäß den folgenden Punkten 8.1 bis 8.9.
8.1 Verfahren zur Herstellung einer *N*-methylsubstituierten Triacetonaminverbindung, dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Strukturen **(I-G)** mit 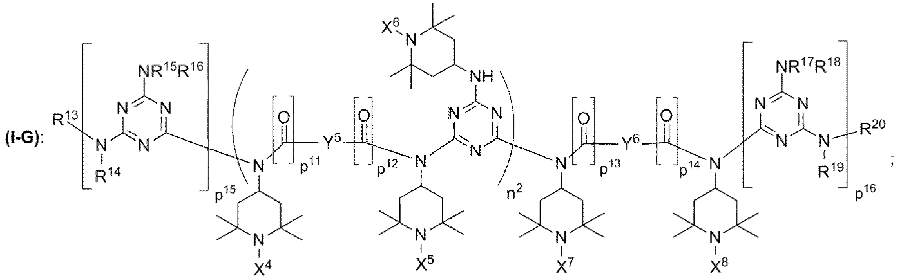
   wobei n² eine ganze Zahl aus dem Bereich 1 bis 20 ist;
   wobei p¹¹, p¹², p¹³, p¹⁴, p¹⁵, p¹⁶ unabhängig voneinander jeweils 0 oder 1 ist;
   wobei X⁴, X⁵, X⁶, X⁷, X⁸ unabhängig voneinander jeweils ausgewählt sind aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y⁵, Y⁶ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i)**, **(ii)** mit 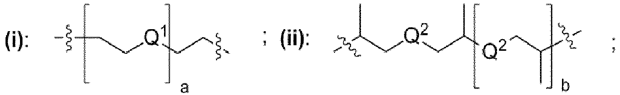 aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
            wobei a eine ganze Zahl ausgewählt aus dem Bereich 1 bis 50 ist,
         wobei b eine ganze Zahl ausgewählt aus dem Bereich 0 bis 50 ist,
   und wobei Y⁵ auch eine direkte Bindung sein kann, falls mindestens einer von p¹¹ und p¹² den Wert 1 aufweist,
   und wobei Y⁶ auch eine direkte Bindung sein kann, falls mindestens einer von p¹³ und p¹⁴ den Wert 1 aufweist;
   wobei die Reste R¹³, R¹⁴, R¹⁵, R¹⁶, R¹⁷, R¹⁸, R¹⁹, R²⁰ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii)**, **(iv)**, **(v)**, **(vi)**, **(vii)**, **(viii)**, **(ix)** mit 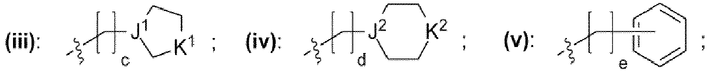 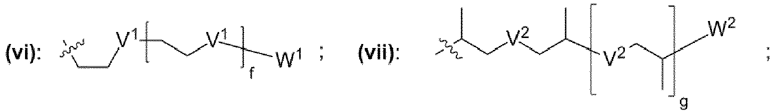 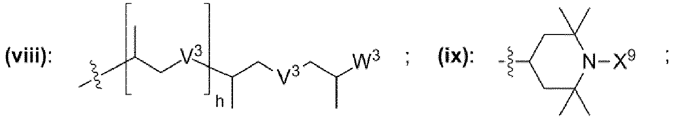 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
         -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
8.2 Verfahren nach Punkt 8.1, wobei p¹¹ = p¹² = p¹³ = p¹⁴ = 0 ist und wobei p¹⁵, p¹⁶ unabhängig voneinander jeweils 0 oder 1 ist.
8.3 Verfahren nach Punkt 8.1 oder 8.2, wobei
   Y⁵, Y⁶ unabhängig voneinander jeweils ausgewählt ist aus der Gruppe bestehend aus unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
8.4 Verfahren nach einem oder mehreren der Punkte 8.1 bis 8.3, wobei die Reste R¹³, R¹⁴, R¹⁵, R¹⁶, R¹⁷, R¹⁸, R¹⁹, R²⁰ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 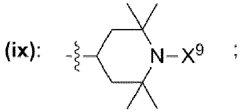 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
8.5 Verfahren nach einem oder mehreren der Punkte 8.1 bis 8.4, wobei die Reste R¹³, R¹⁴, R¹⁵, R¹⁶, R¹⁷, R¹⁸, R¹⁹, R²⁰ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   einem Rest, der eine chemischen Struktur **(ix)** mit 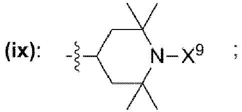 wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
8.6 Verfahren nach einem oder mehreren der Punkte 8.1 bis 8.5, wobei X⁴ = X⁵ = X⁶ = X⁷ = X⁸ = X⁹ = Wasserstoff ist.
8.7 Verfahren nach einem oder mehreren der Punkte 8.1 bis 8.6, wobei Formaldehyd als Gas, als wässrige Lösung oder als Feststoff eingesetzt wird, bevorzugt als wässrige Lösung oder als Feststoff, noch bevorzugter als wässrige Lösung.
8.8 Verfahren nach einem oder mehreren der Punkte 8.1 bis 8.7, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
8.9 Verfahren nach einem oder mehreren der Punkte 8.1 bis 8.8, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem neunten Aspekt ein Verfahren gemäß den folgenden Punkten 9.1 bis 9.8.
9.1 Verfahren zur Herstellung einer *N*-methylsubstituierten Triacetonaminverbindung,
   dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Strukturen **(I-H)** mit 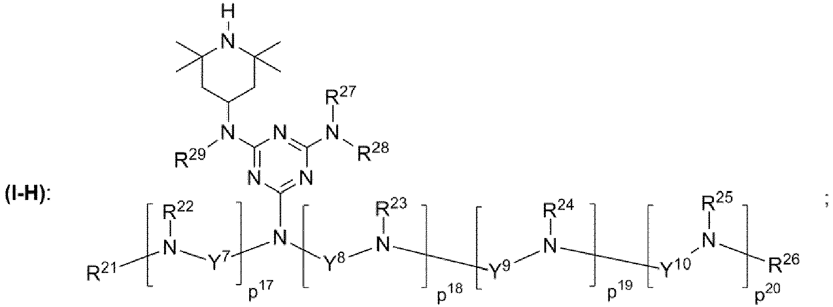
   wobei p¹⁷, p¹⁸, p¹⁹, p²⁰ unabhängig voneinander jeweils 0 oder 1 ist;
   wobei Y⁷, Y⁸, Y⁹, Y¹⁰ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
   zweiwertige Kohlenwasserstoffgruppe mit 6 bis 30 Kohlenstoffatomen, von denen
      mindestens 6 Kohlenstoffatome in einem aromatischen System vorliegen und die übrigen Kohlenstoffatome, falls vorhanden, gesättigt sind,
   ein verbrückender Rest, der eine chemische Struktur ausgewählt aus der Gruppe bestehend aus **(i)**, **(ii)** mit 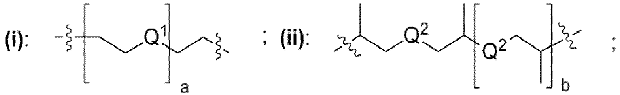 aufweist, wobei
      Q1, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
      wobei a eine ganze Zahl ausgewählt aus dem Bereich 1 bis 50 ist,
      wobei b eine ganze Zahl ausgewählt aus dem Bereich 0 bis 50 ist,
   wobei die Reste R²¹, R²², R²³, R²⁴, R²⁵, R²⁶ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen,
      einer Gruppe mit der chemischen Struktur **(xi)** mit 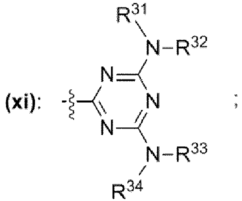 wobei die Reste R²⁷, R²⁸, R²⁹, R³¹, R³², R³³, R³⁴ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(xii)**, **(xiii)**, **(xiv)**, **(xv)**, **(xvi)**, **(xvii)**, **(xviii)** mit 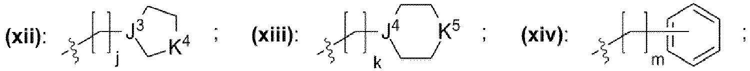 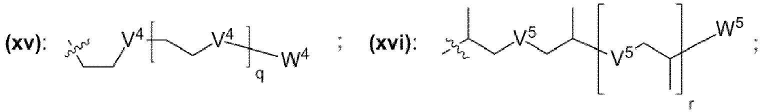 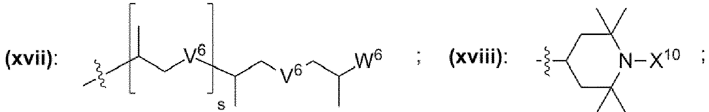 aufweist,
         wobei J³, J⁴ unabhängig voneinander jeweils ausgewählt aus der Gruppe bestehend aus CH, N sind,
         wobei K⁴, K⁵ unabhängig voneinander jeweils ausgewählt aus der Gruppe bestehend aus -O-, -NH-, -N(CH₃)-, -N(CH₂CH₃)-, -S-, -CH₂- sind,
         wobei V⁴, V⁵, V⁶ unabhängig voneinander jeweils ausgewählt aus der Gruppe bestehend aus -O-, -S-, -NH-, -NR"'- mit R'" = unverzweigte oder verzweigte Alkylgruppe mit 1 bis 6 Kohlenstoffatomen sind,
         wobei W⁴, W⁵, W⁶ unabhängig voneinander jeweils ausgewählt aus der Gruppe bestehend aus H, Methyl, Ethyl sind,
         wobei j, k, m, q, r, s unabhängig voneinander jeweils eine ganze Zahl aus dem Bereich 0 bis 50 ist,
         wobei X¹⁰ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
         wobei in den chemischen Strukturen **(xii)**, **(xiii)**, **(xiv)**, **(xv)**, **(xvi)**, **(xvii)**, **(xviii)** mindestens ein an ein Kohlenstoffatom gebundener Wasserstoffrest durch einen Rest ausgewählt aus der Gruppe bestehend
            aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 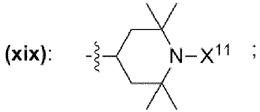 sein können, wobei X¹¹ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
9.2 Verfahren nach Punkt 9.1, wobei
   Y⁷, Y⁸ Y⁹, Y¹⁰ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
9.3 Verfahren nach einem oder mehreren der Punkte 9.1 bis 9.2, wobei die Reste R²¹, R²², R²³, R²⁴, R²⁵, R²⁶ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen,
   einer Gruppe mit der chemischen Struktur **(xi)** mit 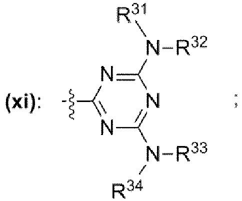 wobei die Reste R²⁷, R²⁸, R²⁹, R³¹, R³², R³³, R³⁴ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur **(xviii)** mit 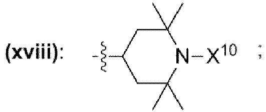 aufweist,
         wobei X¹⁰ ausgewählt ist aus der Gruppe bestehend aus Wasserstoff, -OH, -O-, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 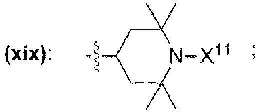
   sein können, wobei X¹¹ ausgewählt ist aus der Gruppe bestehend aus Wasserstoff, OH, -0., unverzweigte oder verzweigte Alkylgruppe mit 1 bis 10 Kohlenstoffatomen, unverzweigte oder verzweigte Alkoxygruppe mit 1 bis 10 Kohlenstoffatomen.
9.4 Verfahren nach einem oder mehreren der Punkte 9.1 bis 9.3, wobei die Reste R²¹, R²², R²³, R²⁴, R²⁵, R²⁶ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   einer Gruppe mit der chemischen Struktur **(xi)** mit 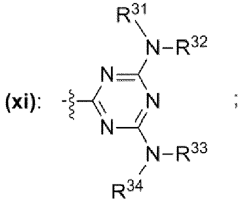 wobei die Reste R²⁷, R²⁸, R²⁹, R³¹, R³², R³³, R³⁴ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   einem Rest, der eine chemischen Struktur **(xviii)** mit 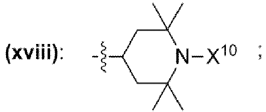 aufweist,
      wobei X¹⁰ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 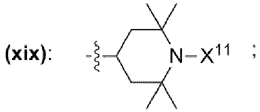 sein können, wobei X¹¹ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
9.5 Verfahren nach einem oder mehreren der Punkte 9.1 bis 9.4, wobei X⁹ = X¹⁰ = X¹¹ = Wasserstoff ist.
9.6 Verfahren nach einem oder mehreren der Punkte 9.1 bis 9.5,
   wobei Formaldehyd als Gas, als wässrige Lösung oder als Feststoff eingesetzt wird, bevorzugt als wässrige Lösung oder als Feststoff, noch bevorzugter als wässrige Lösung.
9.7Verfahren nach einem oder mehreren der Punkte 9.1 bis 9.6, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
9.8 Verfahren nach einem oder mehreren der Punkte 9.1 bis 9.7, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

### Allgemeine Begriffe

Im Sinne der Erfindung ist eine "unverzweigte oder verzweigte Alkylgruppe" ein einwertiger gesättigter Kohlenwasserstoffrest der allgemeinen chemische Struktur **(a)** mit 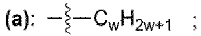 aufweist.

Die Kette an Kohlenstoffatomen "-C_{w}H_{2w+1}" kann dabei linear sein, dann handelt es sich um eine unverzweigte Alkylgruppe. Sie kann aber auch Verzweigungen aufweisen, dann handelt es sich um eine verzweigte Alkylgruppe.

Dabei ist w in der chemischen Struktur **(a)** eine ganze Zahl. w ist bei einer unverzweigten oder verzweigten Alkylgruppe mit 1 bis 30 Kohlenstoffatomen ausgewählt aus dem Bereich 1 bis 30. w ist bei einer unverzweigten oder verzweigten Alkylgruppe mit 1 bis 29 Kohlenstoffatomen ausgewählt aus dem Bereich 1 bis 29. w ist bei einer unverzweigten oder verzweigten Alkylgruppe mit 1 bis 12 Kohlenstoffatomen ausgewählt aus dem Bereich 1 bis 12. w ist bei einer unverzweigten oder verzweigten Alkylgruppe mit 1 bis 10 Kohlenstoffatomen ausgewählt aus dem Bereich 1 bis 10. w ist bei einer unverzweigten oder verzweigten Alkylgruppe mit 1 bis 8 Kohlenstoffatomen ausgewählt aus dem Bereich 1 bis 8. w ist bei einer unverzweigten oder verzweigten Alkylgruppe mit 1 bis 6 Kohlenstoffatomen ausgewählt aus dem Bereich 1 bis 6.

Im Sinne der Erfindung ist eine "unverzweigte oder verzweigte Alkylgruppe mit 1 bis 30 Kohlenstoffatomen" insbesondere ausgewählt aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl, *n*-Nonadecyl, *n*-Eicosyl, *n*-Heneicosyl, *n*-Docosyl, *n*-Tricosyl, *n*-Tetracosyl, *n*-Pentacosyl, *n*-Hexacosyl, *n*-Heptacosyl, *n*-Octocosyl, *n*-Nonacosyl, *n*-Tricontyl.

Im Sinne der Erfindung ist eine "unverzweigte oder verzweigte Alkylgruppe mit 1 bis 12 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl.

Im Sinne der Erfindung ist eine "unverzweigte oder verzweigte Alkylgruppe mit 1 bis 10 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl.

Im Sinne der Erfindung ist eine "unverzweigte oder verzweigte Alkylgruppe mit 1 bis 8 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl.
Im Sinne der Erfindung ist eine "unverzweigte oder verzweigte Alkylgruppe mit 1 bis 6 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl.

Der Begriff "unverzweigte oder verzweigte Alkylengruppe" bezeichnet im Sinne der Erfindung einen zweiwertigen gesättigten Kohlenwasserstoffrest, welcher durch die allgemeine chemische Struktur **(b)** mit 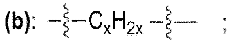 beschrieben werden kann. Die Kette an Kohlenstoffatomen"-CₓH₂ₓ" kann dabei linear sein, dann handelt es sich um eine unverzweigte Alkylengruppe. Sie kann aber auch Verzweigungen aufweisen, dann handelt es sich um eine verzweigte Alkylengruppe. Dabei ist x in der chemischen Struktur **(b)** eine ganze Zahl.

x ist bei einer unverzweigten oder verzweigten Alkylengruppe mit 1 bis 30 Kohlenstoffatomen ausgewählt aus dem Bereich 1 bis 30.

x ist bei einer unverzweigten oder verzweigten Alkylengruppe mit 1 bis 12 Kohlenstoffatomen ausgewählt aus dem Bereich 1 bis 12.

x ist bei einer unverzweigten oder verzweigten Alkylengruppe mit 1 bis 6 Kohlenstoffatomen ausgewählt aus dem Bereich 1 bis 6.

Im Sinne der Erfindung ist eine "zweiwertige gesättigte Kohlenwasserstoffgruppe mit 3 bis 30 Kohlenstoffatomen, welche mindestens einen gesättigten Ring aus 3 bis 30 Kohlenstoffatomen aufweist" insbesondere eine chemische Struktur **(c)** mit 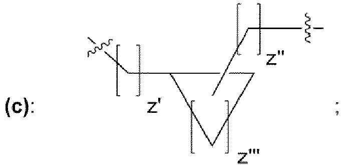 wobei z' eine ganze Zahl zwischen 0 und 27 ist; wobei z" eine ganze Zahl zwischen 0 und 27 ist; wobei z'" eine ganze Zahl zwischen 1 und 28 ist; und wobei gleichzeitig gilt, dass z' + z" + z'" ≤ 28.

Insbesondere ist eine "zweiwertige gesättigte Kohlenwasserstoffgruppe mit 3 bis 30 Kohlenstoffatomen, welche mindestens einen gesättigten Ring aus 3 bis 30 Kohlenstoffatomen aufweist" eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist", noch bevorzugter ""zweiwertige gesättigte Kohlenwasserstoffgruppe mit 3 bis 6 Kohlenstoffatomen, welche mindestens einen gesättigten Ring aus 3 bis 6 Kohlenstoffatomen aufweist".

Eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist" weist im Sinne der Erfindung eine chemische Struktur **(c)** auf, wobei z' eine ganze Zahl zwischen 0 und 9 ist; wobei z" eine ganze Zahl zwischen 0 und 9 ist; wobei z'" eine ganze Zahl zwischen 1 und 10 ist; und wobei gleichzeitig gilt, dass z' + z" + z'" ≤ 10.

Bevorzugt ist eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist" ausgewählt aus der Gruppe bestehend aus Cyclopropylen, Cyclobutylen, Cyclopentylen, Cyclohexylen, Cycloheptylen, Cyclooctylen, Cyclononylen, Cyclodecylen, Cycloundecylen, Cyclododecylen.

Eine "zweiwertige gesättigte Kohlenwasserstoffgruppe mit 3 bis 6 Kohlenstoffatomen, welche mindestens einen gesättigten Ring aus 3 bis 6 Kohlenstoffatomen aufweist" weist im Sinne der Erfindung eine chemische Struktur **(c)** auf, wobei z' eine ganze Zahl zwischen 0 und 3 ist; wobei z" eine ganze Zahl zwischen 0 und 3 ist; wobei z'" eine ganze Zahl zwischen 1 und 4 ist; und wobei gleichzeitig gilt, dass z' + z" + z'" ≤ 4.

Bevorzugt ist eine "zweiwertige gesättigte Kohlenwasserstoffgruppe mit 3 bis 6 Kohlenstoffatomen, welche mindestens einen gesättigten Ring aus 3 bis 6 Kohlenstoffatomen aufweist" ausgewählt aus der Gruppe bestehend aus Cyclopropylen, Cyclobutylen, Cyclopentylen, Cyclohexylen.

Im Sinne der Erfindung ist eine "zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind" insbesondere eine "zweiwertige Kohlenwasserstoffgruppe mit 6 bis 30 Kohlenstoffatomen, von denen 6, 10 oder 14 Kohlenstoffatome in einem aromatischen System vorliegen und die übrigen Kohlenstoffatome, falls vorhanden, gesättigt sind" und bevorzugter ausgewählt aus der Gruppe bestehend aus Naphthylen, Anthrylen, Phenanthrylen sowie der folgenden chemischen Struktur **(d)**: 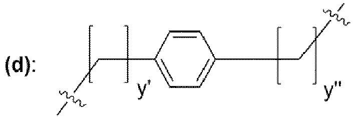 wobei y' eine ganze Zahl zwischen 0 und 24 ist; wobei y" eine ganze Zahl zwischen 0 und 24 ist; und wobei gleichzeitig gilt, dass y' + y" ≤ 24.

Noch bevorzugter handelt es sich dabei um eine "zweiwertige Kohlenwasserstoffgruppe mit 6 bis 30 Kohlenstoffatomen, von denen 6 oder 10 Kohlenstoffatome in einem aromatischen System vorliegen und die übrigen Kohlenstoffatome, falls vorhanden, gesättigt sind" und am bevozugtesten ist diese dann ausgewählt aus der Gruppe bestehend aus Naphthylen, sowie der folgenden chemischen Struktur **(d)**: 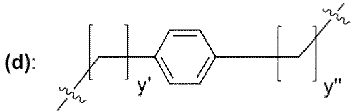 wobei y' eine ganze Zahl zwischen 0 und 24 ist; wobei y" eine ganze Zahl zwischen 0 und 24 ist; und wobei gleichzeitig gilt, dass y' + y" ≤ 24.

Im Sinne der Erfindung ist eine "unverzweigte oder verzweigte Alkoxygruppe" ein organischer Rest der chemischen Struktur 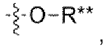 in welchem R** eine unverzweigte oder verzweigte Alkylgruppe ist. Bei einer "unverzweigten oder verzweigten Alkoxygruppe mit 1 bis 30 Kohlenstoffatomen" ist R** eine unverzweigte oder verzweigte Alkylgruppe mit 1 bis 30 Kohlenstoffatomen.

Bei einer "unverzweigten oder verzweigten Alkoxygruppe mit 1 bis 10 Kohlenstoffatomen" ist R** eine unverzweigte oder verzweigte Alkylgruppe mit 1 bis 10 Kohlenstoffatomen.

Im Sinne der Erfindung ist eine "unverzweigte oder verzweigte Alkoxygruppe mit 1 bis 10 Kohlenstoffatomen" insbesondere ausgewählt aus der Gruppe bestehend aus Methoxy, Ethoxy, *n*-Propoxy, *iso*-Propoxy, *n*-Butoxy, *sec*-Butoxy, *iso*-Butoxy, *tert*-Butoxy, *n*-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, *n*-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, *n*-Heptoxy, *n*-Octoxy, *n*-Nonoxy, *n*-Decoxy.

Im Sinne der Erfindung ist eine "unverzweigte oder verzweigte Acylgruppe mit 1 bis 30 Kohlenstoffatomen" ein organischer Rest der chemischen Struktur 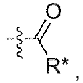 in welchem R* ein unverzweigter oder verzweigter Alkylrest mit 1 bis 29 Kohlenstoffatomen ist.

Insbesondere ist R* ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl, *n*-Nonadecyl, *n*-Eicosyl, *n*-Heneicosyl, *n*-Docosyl, *n*-Tricosyl, *n*-Tetracosyl, *n*-Pentacosyl, *n*-Hexacosyl, *n*-Heptacosyl, *n*-Octocosyl, *n*-Nonacosyl.

"-O·" bezeichnet im Sinne der Erfindung einen radikalischen Sauerstoffrest.

Im Sinne der Erfindung bedeutet die Formulierung "mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann", dass die betreffende Gruppe unsubstituiert vorliegt oder in der betreffenden Gruppe mindestens ein an ein Kohlenstoffatom gebundener Wasserstoffrest, bevorzugt 1 bis 5, noch bevorzugter 1 bis 3, am bevorzugtesten 1 bis 2 an das selbe oder unterschiedliche Kohlenstoffatom(e) gebundene(r) Wasserstoffrest(e), durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) substituiert ist.

### Erfindunqsqemäßes Verfahren

Formaldehyd wird insbesondere im erfindungsgemäßen Verfahren als Gas, als wässrige Lösung oder als Feststoff eingesetzt werden. Bevorzugt wird Formaldehyd im erfindungsgemäßen Verfahren als wässrige Lösung oder als Feststoff (z. B. als Paraformaldehyd) eingesetzt.

In der noch bevorzugteren Ausführungsform, in der Formaldehyd als wässrige Lösung eingesetzt wird, beträgt die Konzentration des Formaldehyds in der Lösung 1.0 bis 37 Gew.-% (w/w, "%" bezieht sich auf das Gewicht des Formaldehyds bezogen auf das Gesamtgewicht der wässrigen Lösung). Bei 37 Gew.-% an Formaldehyd enthalten zum Beispiel 100 g wässrige Lösung 37 g Formaldehyd.

Das erfindungsgemäße Verfahren wird unter reduktiven Bedingungen durchgeführt. Unter "reduktiven Bedingungen" sind die Bedingungen zu verstehen, bei welchen das im Reaktionsschema **<1>** gezeigte Imin durch Wasserstoffanlagerung in das entsprechende Amin überführt wird.

Man stellt im erfindungsgemäßen Verfahren reduktive Bedingungen dadurch ein, dass man die mindestens eine Triacetondiaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu; insbesondere ausgewählt ist aus der Gruppe bestehend aus Ag, Fe, Cr, Mo, Mn, Ni, Co, Cu, Pd, Pt, Ru, Rh; bevorzugt ausgewählt ist aus der Gruppe bestehend aus Ag, Fe, Cr, Ni, Co, Cu, Pd, Pt; bevorzugter ausgewählt ist aus der Gruppe bestehend aus Ag, Fe, Ni, Co, Cu, Pd, Pt; noch bevorzugter ausgewählt ist aus der Gruppe bestehend aus Ag, Fe, Ni, Co, Cu; noch mehr bevorzugter ausgewählt ist aus der Gruppe bestehend Co, Ni; am bevorzugtesten Ni ist.

Die Verwendung eines Vollkatalysators aufweisend mindestens ein Metall **M** ist wesentlich für das erfindungsgemäße Verfahren.

Ein "Vollkatalysator" ist dem Fachmann bekannt und ist ein vollständig von dem katalytischen Material durchdrungener Formkörper. Er unterscheidet sich damit vom "Trägerkatalysator", bei dem die katalytisch aktive Komponente auf einem von der katalytisch aktiven Komponente verschiedenen Träger aufgebracht ist. Ein dem erfindungsgemäßen Verfahren entsprechendes Verfahren, in welchem anstatt eines Vollkatalysators ein Trägerkatalysator eingesetzt wird, beschreibt die Anmeldung EP 3 048 097 A1, welche ein Dokument nach Artikel 54 (3) EPÜ ist. Das mindestens eine Metall **M** im Vollkatalysator liegt insbesondere im elementaren Zustand oder als Verbindung des Metalls **M**, beispielsweise als Oxid, Sulfid, vor, bevorzugt jedoch im elementaren Zustand.

Bevorzugt handelt es sich bei dem Vollkatalysator aufweisend mindestens ein Metall **M** um einen Vollkatalysator aufweisend ein Metall **M** ausgewählt aus Ag, Fe, Ni, Co, Cu, insbesondere Ni, Co, bevorzugt Ni.

Der Vollkatalysator kann eine Legierung des Metalls **M** sein (wobei das Metall **M** zu mindestens > 50 Gew.-% in der Legierung vorliegt, bezogen auf das Gesamtgewicht der Legierung) und beispielsweise außer **M** noch mindestens ein Metall bzw. Halbmetall ausgewählt aus Al, Si, Mg, Zn, Mo, Cr insbesondere AI, aufweisen.

Noch mehr bevorzugter ist der Vollkatalysator aufweisend mindestens ein Metall **M** ausgewählt aus der Gruppe bestehend aus Raney-Kobalt, Raney-Kupfer, Raney-Silber, Raney-Eisen, Raney-Nickel, insbesondere ausgewählt aus Raney-Nickel, Raney-Kobalt, am bevorzugtesten ausgewählt aus Raney-Nickel.

Im Raney-Nickel ist der Anteil des Nickels bezogen auf das Gesamtgewicht des Raney-Nickels insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Nickel insbesondere daneben noch weitere von Nickel verschiedene Metalle und/oder Halbmetalle (wie beispielsweise AI, Mo, Si, Mg, Zn, Mo, Cr) in einer solchen Menge aufweist, dass die Summe der Gewichte aus Nickel und den übrigen Metallen und Halbmetallen 100 Gew.-% ergibt. Das Raney-Nickel kann insbesondere mit Zn, Mo, Cr, bevorzugt Mo dotiert sein, um die katalytischen Eigenschaften zu verbessern.

Im Raney-Kobalt ist der Anteil des Kobalts bezogen auf das Gesamtgewicht des Raney-Kobalts insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Kobalt insbesondere daneben noch weitere von Kobalt verschiedene Metalle und/oder Halbmetalle (wie beispielsweise Al, Mo, Si, Mg, Zn, Mo, Cr) in einer solchen Menge aufweist, dass die Summe der Gewichte aus Kobalt und den übrigen Metallen und Halbmetallen 100 Gew.-% ergibt. Das Raney-Kobalt kann insbesondere mit Zn, Mo, Cr, bevorzugt Mo dotiert sein, um die katalytischen Eigenschaften zu verbessern.

Im Raney-Kupfer ist der Anteil des Kupfers bezogen auf das Gesamtgewicht des Raney-Kupfers insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Kupfer insbesondere daneben noch weitere von Kupfer verschiedene Metalle und/oder Halbmetalle (wie beispielsweise AI, Mo, Si, Mg, Zn, Mo, Cr) in einer solchen Menge aufweist, dass die Summe der Gewichte aus Kupfer und den übrigen Metallen und Halbmetallen 100 Gew.-% ergibt. Das Raney-Kupfer kann insbesondere mit Zn, Mo, Cr, bevorzugt Mo dotiert sein, um die katalytischen Eigenschaften zu verbessern.

Im Raney-Silber ist der Anteil des Silbers bezogen auf das Gesamtgewicht des Raney-Silbers insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Silber insbesondere daneben noch weitere von Silber verschiedene Metalle und/oder Halbmetalle (wie beispielsweise AI, Mo, Si, Mg, Zn, Mo, Cr) in einer solchen Menge aufweist, dass die Summe der Gewichte aus Silber und den übrigen Metallen und Halbmetallen 100 Gew.-% ergibt. Das Raney-Silber kann insbesondere mit Zn, Mo, Cr, bevorzugt Mo dotiert sein, um die katalytischen Eigenschaften zu verbessern.

Im Raney-Eisen ist der Anteil des Eisens bezogen auf das Gesamtgewicht des Raney-Eisens insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Eisen insbesondere daneben noch weitere von Eisen verschiedene Metalle und/oder Halbmetalle (wie beispielsweise AI, Mo, Si, Mg, Zn, Mo, Cr) in einer solchen Menge aufweist, dass die Summe der Gewichte aus Eisen und den übrigen Metallen und Halbmetallen 100 Gew.-% ergibt. Das Raney-Eisen kann insbesondere mit Zn, Mo, Cr, bevorzugt Mo dotiert sein, um die katalytischen Eigenschaften zu verbessern.

Die Herstellung der erfindungsgemäßen Vollkatalysatoren ist dem Fachmann bekannt. Die Herstellung des Raney-Nickels ist zum Beispiel in den US 1,629,190, DE 20 2010 007837 U1 beschrieben. Dazu legiert man Ni mit Al, Si, Mg oder Zn (insbesondere mit Al, bevorzugt im Verhältnis 1:1) und löst aus der Legierung nach mechanischer Zerkleinerung mit Alkalien (insbesondere NaOH) das katalytisch unwirksame Metall (Al) mindestens teilweise heraus.

Entsprechend werden auch Raney-Kupfer, Raney-Kobalt, Raney Silber oder Raney-Eisen hergestellt (beschrieben zum Beispiel in der DE 20 2010 007837 U1).

Ohne einen solchen Vollkatalysator würde man nur unerwünschte Produkte erhalten. C. Harries beschreibt zum Beispiel auf Seiten 220 bis 222 seines Artikels "Untersuchungen über die cyclischen Acetonbasen" in Justus Liebigs Annalen der Chemie, Band 417, 1918, Seiten 107 bis 191 eine Umsetzung von 4-Amino-2,2,6,6-Tetramethylaminopiperidin mit Acetanhydrid ohne Vollkatalysator (oder Trägerkatalysator), was zu hohen Ausbeuten an der entsprechenden, hier unerwünschten Amidverbindung führt.

Das erfindungsgemäße Verfahren kann man lösungsmittelfrei oder auch in mindestens einem Lösungsmittel durchführen, bevorzugt in mindestens einem Lösungsmittel. Als Lösungsmittel eignen sich dabei alle Lösungsmittel, in denen sich die Reaktanden gut lösen und welche auch keinen störenden Einfluss auf das erfindungsgemäße Verfahren haben. Insbesondere ist das Lösungsmittel ausgewählt aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser; bevorzugt ist das Lösungsmittel ausgewählt aus der Gruppe bestehend aus aliphatische Lösungsmittel, aromatische Lösungsmittel, Ether, Alkohole, Wasser; bevorzugter ist das Lösungsmittel ausgewählt aus der Gruppe bestehend aus aliphatische Lösungsmittel, aromatische Lösungsmittel, Ether, Alkohole, Wasser; noch bevorzugter ist das Lösungsmittel ausgewählt aus der Gruppe bestehend aus aliphatische Lösungsmittel, aromatische Lösungsmittel, Alkohole, Wasser. Das Lösungsmittel ist besonders bevorzugt ausgewählt aus aromatischen Lösungsmittel (insbesondere Toluol), Alkohole (insbesondere Methanol).

Aliphatische Lösungsmittel sind insbesondere ausgewählt aus der Gruppe bestehend aus Pentan, Hexan, Heptan, Octan, Decan, Cyclopentan, Cyclohexan, Methylcyclohexan, Petrolether.

Aromatische Lösungsmittel sind insbesondere ausgewählt aus der Gruppe bestehend aus Benzol, Toluol, Xylol, Ethylbenzol, Cumol, Brombenzol, Chlorbenzol, Dichlorbenzol, Furan, bevorzugt Toluol.

Ether sind insbesondere ausgewählt aus der Gruppe bestehend aus Diethylether, Dipropylether, Dibutylether, Methyl-*tert*-Butylether, Ethylenglykolmonomethylether, Ethylenglykolmonoethylether, Ethylenglykoldimethylether, Ethylenglykoldiethylether, Diethylenglykolmonomethylether, Diethylenglykolmonoethylether, Diethylenglykoldimethylether, Diethylenglykoldiethylether, Triethylenglykolmonomethylether, Triethylenglykolmonoethylether, Triethylenglykoldimethylether, Triethylenglykoldiethylether, Polyethylenglykolmonomethylether, Polyethylenglykolmonoethylether, Polyethylenglykoldimethylether, Polyethylenglykoldiethylether, 1,4-Dioxan, 1,3-Dioxan, Tetrahydrofuran.

Halogenierte Lösungsmittel sind insbesondere ausgewählt aus der Gruppe bestehend aus Dichlormethan, Chloroform, Tetrachlormethan.

Amide sind insbesondere ausgewählt aus der Gruppe bestehend aus Dimethylformamid, Dimethylacetamid.

Thioverbindungen sind insbesondere ausgewählt aus der Gruppe bestehend aus Dimethylsulfoxid, Sulfolan.

Carbonsäuren sind insbesondere ausgewählt aus der Gruppe bestehend aus Ameisensäure, Essigsäure, Propionsäure, Butansäure, Pentansäure.

Alkohole, sind insbesondere ausgewählt aus der Gruppe bestehend aus Methanol, Ethanol, Propanol, *iso*-Propanol, 1,2-Propandiol, 1,3-Propandiol, Glycerin, Butanol, *sec*-Butanol, *iso*-Butanol, *tert*-Butanol, 1,2-Butandiol, 1,3-Butandiol, 1,4-Butandiol, 1-Pentanol, 2-Pentanol, 3-Pentanol, *tert*-Amylalkohol, 1,2-Pentandiol, 1,3-Pentandiol, 1,4-Pentandiol, 1,5-Pentandiol, Cyclopentanol, Hexanol, Cyclohexanol Heptanol, Octanol, Nonanol, Decanol, Ethylenglykol, Diethylenglykol, Triethylenglykol, Polyethylenglykol, Benzylalkohol, Phenol; bevorzugt ausgewählt aus Methanol, Ethanol, *n*-Propanol, *iso*-Propanol; bevorzugt Methanol.

Das erfindungsgemäße Verfahren lässt sich kontinuierlich oder nicht kontinuierlich, also batchweise, durchführen.

Die Reaktionszeit hängt vom Fortgang des Verfahrens und vom gewünschten Umsatz ab - üblicherweise strebt man einen größtmöglichen Umsatz an und führt das erfindungsgemäße Verfahren so lange fort, bis kein Eduktumsatz mehr beobachtet werden kann.

Die Temperatur beim erfindungsgemäßen Verfahren liegt bevorzugt im Bereich von 20°C bis 350°C, bevorzugter im Bereich von 50°C bis 300°C, noch bevorzugter im Bereich von 50°C bis 250°C, am bevorzugtesten im Bereich von 70°C bis 200°C, noch bevorzugter bei 80°C bis 140°C.

Der Druck beim erfindungsgemäßen Verfahren liegt bevorzugt im Bereich von 2 bar bis 500 bar, bevorzugter im Bereich von 5 bar bis 350 bar, noch bevorzugter im Bereich von 15 bar bis 300 bar, noch bevorzugter 20 bis 42 bar.

Die obigen Temperaturbereiche und Druckbereiche können natürlich auch in Kombination vorliegen. So kann man das Verfahren bevorzugt bei einer Temperatur im Bereich von 20°C bis 350°C, [bevorzugter im Bereich von 50°C bis 300°C, noch bevorzugter im Bereich von 50°C bis 250°C, am bevorzugtesten im Bereich von 70°C bis 200°C, noch bevorzugter bei 80°C - 140°C] und einem Druck im Bereich von 2 bar bis 500 bar [bevorzugt im Bereich von 2 bar bis 500 bar, bevorzugter im Bereich von 5 bar bis 350 bar, noch bevorzugter im Bereich von 15 bar bis 300 bar, noch bevorzugter 20 bis 42 bar] durchführen.

Dieses Verfahren löst die erfindungsgemäßen Aufgaben. Besonders vorteilhaft ist, dass bei der Reaktion lediglich Wasser als Nebenprodukt entsteht. Zudem kann der geringe Überschuss an Formaldehyd entweder durch Hydrierung in Methanol überführt werden oder destillativ abgetrennt und gegebenenfalls zurückgeführt werden.

Die Aufarbeitung des Rohproduktes ist daher sehr einfach: Der Katalysator wird durch Filtration entfernt (vorstellbar wäre technisch auch die Verwendung eines Festbettkatalysators, so dass auch dieser Schritt entfallen würde), anschließend wird das Rohprodukt destillativ aufgereinigt. Es fallen bei der Destillation lediglich Methanol (kann recyclelt werden), Wasser, das Produkt und gegebenenfalls Formaldehyd (kann ebenfalls recyclelt werden) an.

Im Gegensatz zu den Verfahren nach dem Stand der Technik muss also keine Abtrennung von den gebildeten Salzen erfolgen (erfolgt im Allgemeinen durch Extraktion mit einem zusätzlichen Lösungsmittel), zudem fallen die genannten Salze (bzw. deren wässrige Lösung) nicht als Abfallstrom an.

Zusätzlich vorteilhaft ist bei dem erfindungsgemäßen Verfahren, dass zusätzlich zur Einführung der Methylgruppe am Piperidinstickstoff (*N¹*) auch andere Aminogruppen simultan methyliert werden können. Zum Beispiel können *N*⁴-Alkyl-4-amino-2,2,6,6-tetramethylpiperidine selektiv in die *N¹*,*N⁴*-Dimethyl-*N⁴*-alkyl-4-amino-2,2,6,6-tetramethylpiperidine überführt werden. In diesem Fall werden also zwei oder mehr Methylgruppen simultan eingeführt.

Ein weiterer Vorteil besteht darin, dass auch die Anwesenheit von tertiären Aminogruppen toleriert wird, ohne dass diese in die quartären Ammoniumsalze überführt werden.

Die nachfolgenden Beispiele sollen die Erfindung näher erläutern.

### Beispiele

37 Gew.-%-ige Formalinlösung ist erhältlich von der Firma Sigma Aldrich.

*n*-Butyl-Triacetondiamin ist erhältlich von der Firma Evonik Industries AG.

Raney-Nickel sowie die weiteren Vollkatalysatoren Ru, Pt, Rh, Pd, Ir und Co sind erhältlich von der Firma Sigma-Aldrich oder der Firma Strem.

### Erfindungsgemäße Beispiele E1 - E7

In einem 100 ml - Druckautoklav werden 53 g *n*-Butyl-Triacetondiamin (0.25 mol), 25 ml Toluol und 0.5 mmol Raney-Nickel (**E1**), Ru (**E2**), Pt (**E3**), Rh (**E4**), Pd (**E5**), Ir (**E6**) bzw. Co (**E7**) gegeben. Im Anschluss wird eine wässrige Lösung umfassend 0.25 mol Formaldehyd (37 Gew.-% an Formaldehyd, wobei die %-Angabe bezogen auf das Gesamtgewicht der Lösung zu verstehen ist, d.h. 100 g Lösung enthalten 37 g Formaldehyd) hinzugefügt und der Reaktor verschlossen.

Unter Rühren wird Wasserstoff aufgedrückt (10 bar H₂) und die Autoklaveninnentemperatur innerhalb von 1 Stunde von 60 °C bis 90 °C erhöht, danach 3 Stunden bei 90 °C gehalten. Im Anschluss daran wird nochmal 1 Stunde lang bei 120 °C umgesetzt.

Der Reaktor wird danach abgekühlt und entspannt. Das Rohprodukt wird ausgetragen, filtriert und anschließend zunächst das Lösungsmittel entfernt (80 - 120 °C, 400 mbar). Der Rückstand wird anschließend mittels einer Vakuumdestillation unter Verwendung einer 0.5 m Füllkörperkolonne gereinigt.

Dann wird per Gaschromatographie (= GC, zum Beispiel mit einem Agilent 5890 bzw. 7890, FID-Detektor) die Ausbeute an Produkt und gegebenenfalls die Ausbeute des/ der Nebenprodukte(s) im erhaltenen Rohprodukt bestimmt

Die Ergebnisse sind wie folgt:

| Versuch | Ausbeute an *N⁴-*Monomethyl-Butyl-TAD (in %) | Ausbeute an *N*¹,*N⁴*-DimethylButyl-TAD (in %) |
|---|---|---|
| **E1** | 87 | 8 |
| **E2** | 82 | 11 |
| **E3** | 84 | 10 |
| **E4** | 88 | 6 |
| **E5** | 85 | 13 |
| **E6** | 79 | 4 |
| **E7** | 62 | 9 |

Die Ergebnisse zeigen überraschend, dass mit dem neuartigen Verfahren eine selektive Methylierung am *N⁴*-Stickstoffatom mit hoher Ausbeute möglich ist.
*N¹* bezeichnet bei *n*-Butyl-Triacetondiamin das Stickstoffatom innerhalb des 2,2,6,6-Tetramethylpiperidinringes.
*N⁴* bezeichnet bei *n*-Butyl-Triacetondiamin das Stickstoffatom außerhalb des 2,2,6,6-Tetramethylpiperidinringes.

Weiterhin überraschend ist die besonders gute Selektivität bei Rh (**E4**) und Ni (**E1**).

## Patentansprüche

1. Verfahren zur Herstellung einer *N*-methylsubstituierten Triacetonaminverbindung,
**dadurch gekennzeichnet, dass** man mindestens eine Triacetonaminverbindung **(I)** mit Formaldehyd unter reduktiven Bedingungen umsetzt,
wobei die Triacetonaminverbindung **(I)** ausgewählt ist aus der Gruppe bestehend aus den chemischen Strukturen **(I-A)**, **(I-B)**, **(I-C)**, **(I-D)**, **(I-E)**, **(I-F)**, **(I-G)**, **(I-H)** mit 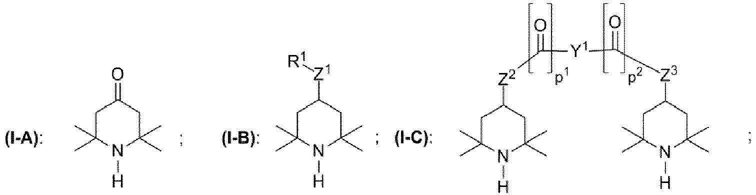 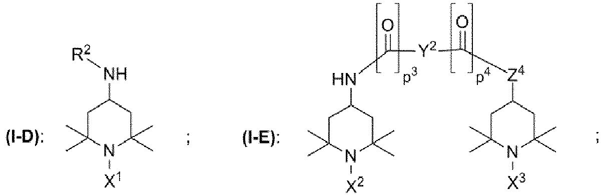 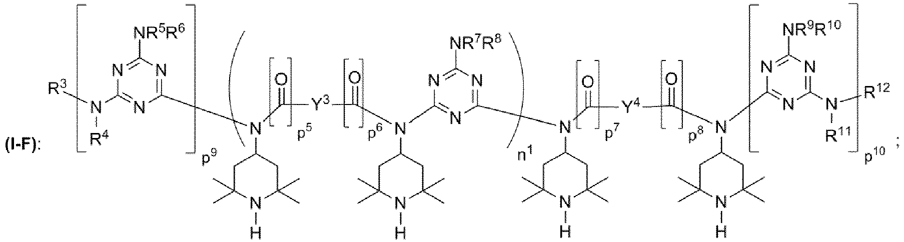 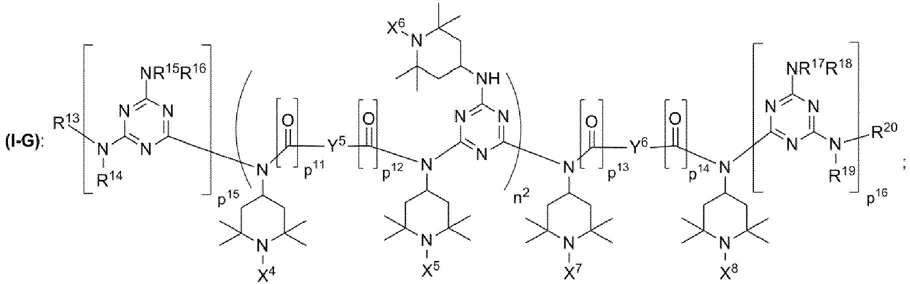 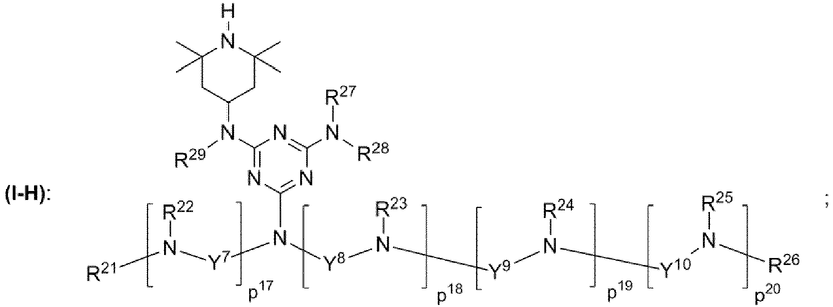
wobei n¹, n² unabhängig voneinander jeweils eine ganze Zahl aus dem Bereich 1 bis 20 ist;
wobei p¹ p², p³, p⁴, p⁵, p⁶, p⁷, p⁸, p⁹, p¹⁰, p¹¹, p¹², p¹³, p¹⁴, p¹⁵, p¹⁶, p¹⁷, p¹⁸, p¹⁹, p²⁰ unabhängig voneinander jeweils 0 oder 1 ist;
wobei X¹ ausgewählt ist aus der Gruppe bestehend aus OH, -0., unverzweigte oder verzweigte Alkylgruppe mit 1 bis 10 Kohlenstoffatomen, unverzweigte oder verzweigte Alkoxygruppe mit 1 bis 10 Kohlenstoffatomen;
wobei X², X³ ausgewählt sind aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen, und wobei X², X³ jeweils unabhängig voneinander ausgewählt sind, bis auf die Ausnahme, dass nicht gilt: X² = X³ = Wasserstoff;
wobei X⁴, X⁵, X⁶, X⁷, X⁸ unabhängig voneinander jeweils ausgewählt sind aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
wobei Y¹, Y², Y³, Y⁴, Y⁵, Y⁶, Y⁷, Y⁸, Y⁹, Y¹⁰ unabhängig voneinander jeweils ausgewählt ist aus der Gruppe bestehend aus
unverzweigte oder verzweigte Alkylengruppe mit 1 bis 30 Kohlenstoffatomen,
zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
ein verbrückender Rest, der eine chemische Struktur ausgewählt aus der Gruppe bestehend aus **(i)**, **(ii)** mit 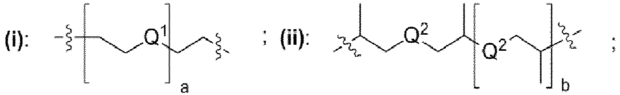 aufweist, wobei
Q1, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
wobei a eine ganze Zahl ausgewählt aus dem Bereich 1 bis 50 ist,
wobei b eine ganze Zahl ausgewählt aus dem Bereich 0 bis 50 ist,
und wobei Y¹ auch eine direkte Bindung sein kann, falls mindestens einer von p¹ und p² den Wert 1 aufweist,
und wobei Y² auch eine direkte Bindung sein kann, falls mindestens einer von p³ und p⁴ den Wert 1 aufweist,
und wobei Y³ auch eine direkte Bindung sein kann, falls mindestens einer von p⁵ und p⁶ den Wert 1 aufweist,
und wobei Y⁴ auch eine direkte Bindung sein kann, falls mindestens einer von p⁷ und p⁸ den Wert 1 aufweist,
und wobei Y⁵ auch eine direkte Bindung sein kann, falls mindestens einer von p¹¹ und p¹² den Wert 1 aufweist,
und wobei Y⁶ auch eine direkte Bindung sein kann, falls mindestens einer von p¹³ und p¹⁴ den Wert 1 aufweist;
und wobei Z¹, Z², Z³, Z⁴ unabhängig voneinander jeweils ausgewählt sind aus der Gruppe bestehend aus -O-, -S-, -NR³⁰-;
wobei die Reste R¹, R², R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(C H₃)(CH₂CH₃) ersetzt sein kann,
einem Rest, der eine chemischen Struktur ausgewählt aus der Gruppe bestehend aus **(iii)**, **(iv)**, **(v)**, **(vi)**, **(vii)**, **(viii)**, **(ix)** mit 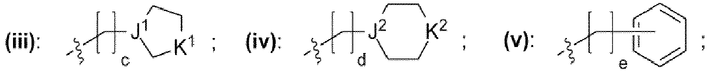 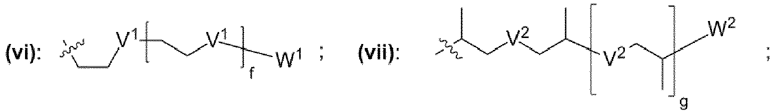 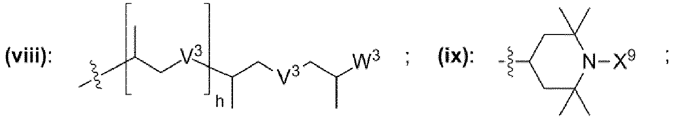 aufweist,
wobei J¹, J² unabhängig voneinander jeweils ausgewählt aus der Gruppe bestehend aus CH, N sind,
wobei K¹, K² unabhängig voneinander jeweils ausgewählt aus der Gruppe bestehend aus -O-, -NH-, -N(CH₃)-, -N(CH₂CH₃)-, -S-, -CH₂- sind,
wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
wobei W¹, W², W³ unabhängig voneinander jeweils ausgewählt aus der Gruppe bestehend aus H, Methyl, Ethyl sind,
wobei c, d, e, f, g, h unabhängig voneinander jeweils eine ganze Zahl aus dem Bereich 0 bis 50 ist,
wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
wobei in den chemischen Strukturen **(iii)**, **(iv)**, **(v)**, **(vi)**, **(vii)**, **(viii)**, **(ix)** mindestens ein an ein Kohlenstoffatom gebundener Wasserstoffrest durch einen Rest ausgewählt aus der Gruppe bestehend aus
-OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
wobei die Reste R⁷, R⁸ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
und wobei, wenn p⁹ = 1, -NR³R⁴ auch ein Rest der chemischen Struktur **(x)** sein kann,
und wobei, wenn p¹⁰ = 1, -NR¹¹R¹² auch ein Rest der chemischen Struktur **(x)** sein kann,
und wobei die Reste -NR⁵R⁶, -NR⁷R⁸, -NR⁹R¹⁰ unabhängig voneinander jeweils auch ein Rest der chemischen Struktur **(x)** sein können,
wobei die chemische Struktur **(x)** wie folgt definiert ist: 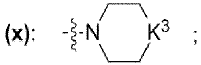 wobei K³ ausgewählt ist aus der Gruppe bestehend aus -O-, -S-, -NH-, -N(CH₃)-, -N(CH₂CH₃)-;
wobei die Reste R²¹, R²², R²³, R²⁴, R²⁵, R²⁶ unabhängig voneinander jeweils ausgewählt sind aus der Gruppe bestehend aus
Wasserstoff,
unverzweigte oder verzweigte Alkylgruppe mit 1 bis 30 Kohlenstoffatomen, einer Gruppe mit der chemischen Struktur **(xi)** mit 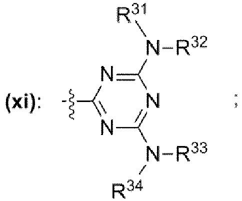 wobei die Reste R²⁷, R²⁸, R²⁹, R³¹, R³², R³³, R³⁴ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur ausgewählt aus der Gruppe bestehend aus **(xii)**, **(xiii)**, **(xiv)**, **(xv)**, **(xvi)**, **(xvii)**, **(xviii)** mit 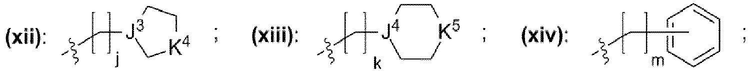 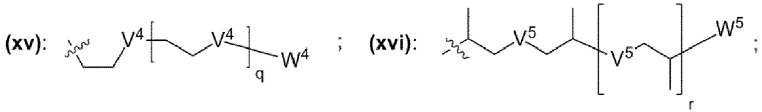 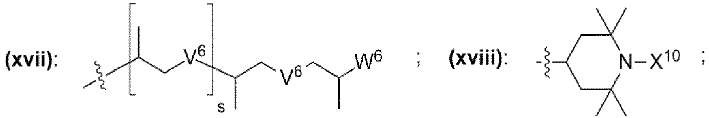 aufweist,
wobei J³, J⁴ unabhängig voneinander jeweils ausgewählt aus der Gruppe bestehend aus CH, N sind,
wobei K⁴, K⁵ unabhängig voneinander jeweils ausgewählt aus der Gruppe bestehend aus -O-, -NH-, -N(CH₃)-, -N(CH₂CH₃)-, -S-, -CH₂- sind, wobei V⁴, V⁵, V⁶ unabhängig voneinander jeweils ausgewählt aus der Gruppe bestehend aus -O-, -S-, -NH-, -NR"'- mit R'" = unverzweigte oder verzweigte Alkylgruppe mit 1 bis 6 Kohlenstoffatomen sind,
wobei W⁴, W⁵, W⁶ unabhängig voneinander jeweils ausgewählt aus der Gruppe bestehend aus H, Methyl, Ethyl sind,
wobei j, k, m, q, r, s unabhängig voneinander jeweils eine ganze Zahl aus dem Bereich 0 bis 50 ist,
wobei X¹⁰ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
wobei in den chemischen Strukturen **(xii)**, **(xiii)**, **(xiv)**, **(xv)**, **(xvi)**, **(xvii)**, **(xviii)** mindestens ein an ein Kohlenstoffatom gebundener Wasserstoffrest durch einen Rest ausgewählt aus der Gruppe bestehend
aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 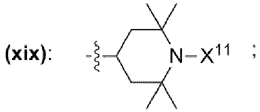 sein können, wobei X¹¹ ausgewählt ist aus der Gruppe bestehend aus Wasserstoff, OH, -0., unverzweigte oder verzweigte Alkylgruppe mit 1 bis 10 Kohlenstoffatomen, unverzweigte oder verzweigte Alkoxygruppe mit 1 bis 10 Kohlenstoffatomen,
und wobei man reduktive Bedingungen dadurch einstellt, dass man die mindestens eine Triacetonaminverbindung **(I)** mit Formaldehyd in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu.

2. Verfahren nach Anspruch 1, wobei p¹ = p² = p³ = p⁴ = p⁵ = p⁶ = p⁷ = p⁸ = p¹¹ = p¹² = p¹³ = p¹⁴ = 0 ist und wobei p⁹, p¹⁰, p¹⁵, p¹⁶, p¹⁷, p¹⁸, p¹⁹, p²⁰ unabhängig voneinander jeweils 0 oder 1 ist.

3. Verfahren nach Anspruch 1 oder 2, wobei
Y¹, Y², Y³, Y⁴, Y⁵, Y⁶, Y⁷, Y⁸, Y⁹, Y¹⁰ unabhängig voneinander jeweils ausgewählt ist aus der Gruppe bestehend aus
unverzweigte oder verzweigte Alkylengruppe mit 1 bis 30 Kohlenstoffatomen,
zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.

4. Verfahren nach einem oder mehreren der Ansprüche 1 bis 3, wobei die Reste R¹, R², R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur **(ix)** mit 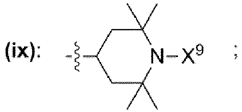 aufweist,
wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
wobei die Reste R⁷, R⁸ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
und wobei, wenn p⁹ = 1, -NR³R⁴ auch ein Rest der chemischen Struktur **(x)** sein kann,
und wobei, wenn p¹⁰ = 1, -NR¹¹R¹² auch ein Rest der chemischen Struktur **(x)** sein kann,
und wobei die Reste -NR⁵R⁶, -NR⁷R⁸, -NR⁹R¹⁰ unabhängig voneinander jeweils auch ein Rest der chemischen Struktur **(x)** sein können,
wobei die chemische Struktur **(x)** wie folgt definiert ist: 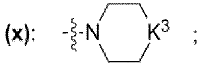 wobei K³ ausgewählt ist aus der Gruppe bestehend aus -O-, -S-, -NH-, -N(CH₃)-, -N(CH₂CH₃)-;
wobei die Reste R²¹, R²², R²³, R²⁴, R²⁵, R²⁶ unabhängig voneinander jeweils ausgewählt sind aus der Gruppe bestehend aus
Wasserstoff,
unverzweigte oder verzweigte Alkylgruppe mit 1 bis 30 Kohlenstoffatomen,
einer Gruppe mit der chemischen Struktur **(xi)** mit 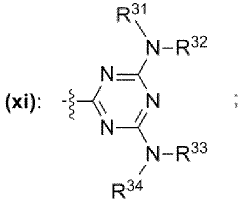 wobei die Reste R²⁷, R²⁸, R²⁹, R³¹, R³², R³³, R³⁴ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur **(xviii)** mit 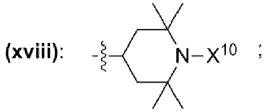 aufweist,
wobei X¹⁰ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 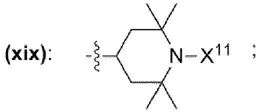 sein können, wobei X¹¹ ausgewählt ist aus der Gruppe bestehend aus Wasserstoff, OH, -O·, unverzweigte oder verzweigte Alkylgruppe mit **1** bis 10 Kohlenstoffatomen, unverzweigte oder verzweigte Alkoxygruppe mit 1 bis 10 Kohlenstoffatomen.

5. Verfahren nach einem oder mehreren der Ansprüche 1 bis 4, wobei
X⁴ = X⁵ = X⁶ = X⁷ = X⁸ = X⁹ = X¹⁰ = X¹¹ = Wasserstoff ist.

6. Verfahren nach einem oder mehreren der Ansprüche 1 bis 5, wobei die Triacetonaminverbindung **(I)** ausgewählt ist aus der Gruppe bestehend aus den chemischen Strukturen **(I-A), (I-B), (I-C), (I-D), (I-E).**

7. Verfahren nach einem oder mehreren der Ansprüche 1 bis 6, wobei die Triacetonaminverbindung **(I)** ausgewählt ist aus der Gruppe bestehend aus den chemischen Strukturen **(I-A), (I-B), (I-D),**
und wobei Z¹ ausgewählt ist aus der Gruppe bestehend aus -O-, -S-, -NR³⁰-;
wobei die Reste R¹, R^{2,} R³⁰ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.

8. Verfahren nach einem oder mehreren der Ansprüche 1 bis 7, wobei die Triacetonaminverbindung **(I)** ausgewählt ist aus der Gruppe bestehend aus den chemischen Strukturen **(I-A), (I-B), (I-D)**
und wobei Z¹ ausgewählt ist aus der Gruppe bestehend aus -O-, -NR³⁰-;
wobei die Reste R¹, R², R³⁰ unabhängig voneinander jeweils ausgewählt sind aus der Gruppe bestehend aus
Wasserstoff,
unverzweigte oder verzweigte Alkylgruppe mit 1 bis 12 Kohlenstoffatomen.

9. Verfahren nach einem oder mehreren der Ansprüche 1 bis 8, wobei Formaldehyd als Gas, als wässrige Lösung oder als Feststoff eingesetzt wird.

10. Verfahren nach einem oder mehreren der Ansprüche 1 bis 9, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.

11. Verfahren nach einem oder mehreren der Ansprüche 1 bis 10, wobei man die mindestens eine Triacetonaminverbindung (I) mit Formaldehyd unter reduktiven Bedingungen bei einer Temperatur im Bereich von 20 °C bis 350 °C und einem Druck im Bereich von 2 bar bis 500 bar umsetzt.

12. Verfahren nach einem oder mehreren der Ansprüche 1 bis 11, wobei das Metall M ausgewählt ist aus der Gruppe bestehend aus Ni, Co, Cu, Fe, Ag.

## Claims

1. Process for preparing an N-methyl-substituted triacetonamine compound,
**characterized in that** at least one triacetonamine compound **(I)** is reacted with formaldehyde under reductive conditions,
where the triacetonamine compound **(I)** is selected from the group consisting of the chemical structures **(I-A), (I-B), (I-C), (I-D), (I-E), (I-F), (I-G), (I-H)** with 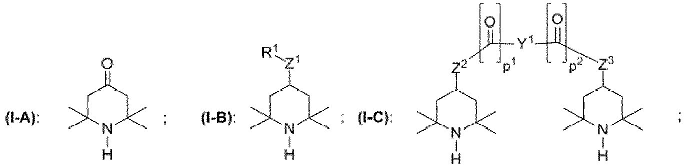 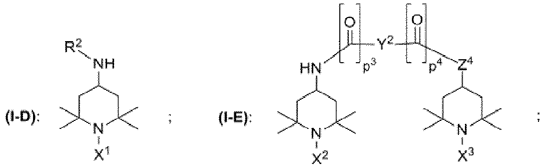 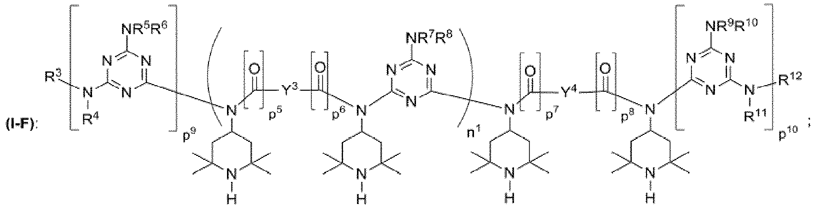 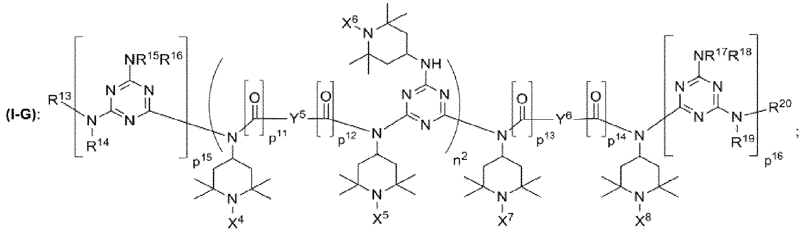 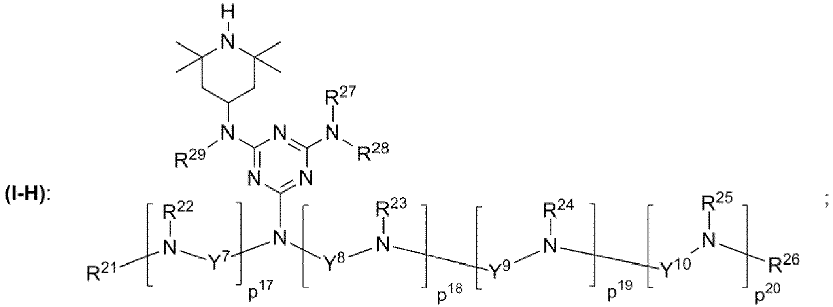
where n¹, n² are each independently an integer from the range of 1 to 20;
where p¹, p², p³, p⁴, p⁵, p⁶, p⁷, p⁸, p⁹, p¹⁰, p¹¹, p¹², p¹³, p¹⁴, p¹⁵, p¹⁶, p¹⁷, p¹⁸, p^{19,} p²⁰ are each independently 0 or 1;
where X¹ is selected from the group consisting of OH, - O·, unbranched or branched alkyl group having 1 to 10 carbon atoms, unbranched or branched alkoxy group having 1 to 10 carbon atoms;
where X², X³ are selected from the group consisting of hydrogen, OH, -O·, unbranched or branched alkyl group having 1 to 10 carbon atoms, unbranched or branched alkoxy group having 1 to 10 carbon atoms, and where X², X³ are each selected independently, with the exclusion of: X² = X³ = hydrogen;
where X⁴, X⁵, X⁶, X⁷, X⁸ are each independently selected from the group consisting of hydrogen, OH, -O·, unbranched or branched alkyl group having 1 to 10 carbon atoms, unbranched or branched alkoxy group having 1 to 10 carbon atoms;
where Y¹, Y², Y³, Y⁴, Y⁵, Y⁶, Y⁷, Y⁸, Y⁹, Y¹⁰ are each independently selected from the group consisting of
unbranched or branched alkylene group having 1 to 30 carbon atoms,
divalent saturated hydrocarbyl group having 3 to 30 carbon atoms and having at least one saturated ring composed of 3 to 30 carbon atoms,
divalent hydrocarbyl group having 6 to 30 carbon atoms, of which at least 6 carbon atoms are present in an aromatic system and the other carbon atoms, if present, are saturated,
a bridging radical having a chemical structure selected from the group consisting of **(i), (ii)** with 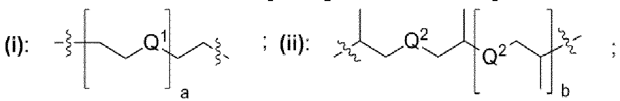 where
Q¹, Q² are each independently selected from the group consisting of -O-, -S-, -NH- and -NR'-with R' = unbranched or branched alkyl group having 1 to 6 carbon atoms,
where a is an integer selected from the range of 1 to 50,
where b is an integer selected from the range of 0 to 50,
and where Y¹ may also be a direct bond if at least one of p¹ and p² has the value of 1,
and where Y² may also be a direct bond if at least one of p³ and p⁴ has the value of 1,
and where Y³ may also be a direct bond if at least one of p⁵ and p⁶ has the value of 1,
and where Y⁴ may also be a direct bond if at least one of p⁷ and p⁸ has the value of 1,
and where Y⁵ may also be a direct bond if at least one of p¹¹ and p¹² has the value of 1,
and where Y⁶ may also be a direct bond if at least one of p¹³ and p¹⁴ has the value of 1;
and where Z¹, Z², Z³, Z⁴ are each independently selected from the group consisting of -O-, -S-, -NR³⁰- ;
where the R¹, R^{2,} R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) ,
unbranched or branched acyl group which has 1 to 30 carbon atoms and in which at least one hydrogen radical may be replaced by a radical selected from the group consisting of
-OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) ,
a radical having a chemical structure selected from the group consisting of **(iii), (iv), (v), (vi), (vii), (viii), (ix)** with 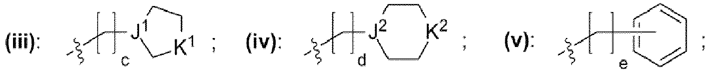 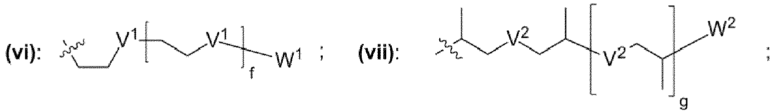 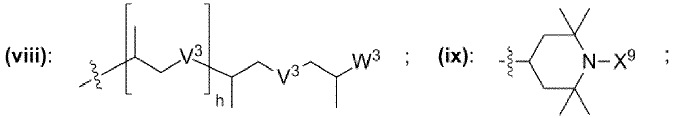
where J¹, J² are each independently selected from the group consisting of CH, N,
where K¹, K² are each independently selected from the group consisting of -O-, -NH-, -N(CH₃)-, -N(CH₂CH₃)-, -S-, -CH₂-,
where V¹, V², V³ are each independently selected from the group consisting of -O-, -S-, -NH-, - NR"- with R" = unbranched or branched alkyl group having 1 to 6 carbon atoms,
where W¹, W², W³ are each independently selected from the group consisting of H, methyl, ethyl,
where c, d, e, f, g, h are each independently an integer from the range of 0 to 50,
where X⁹ is selected from the group consisting of hydrogen, -OH, -O., unbranched or branched alkyl group having 1 to 10 carbon atoms, unbranched or branched alkoxy group having 1 to 10 carbon atoms,
where, in the chemical structures **(iii), (iv)**, **(v), (vi), (vii), (viii), (ix),** at least one hydrogen radical bonded to a carbon atom may be replaced by a radical selected from the group consisting of
-OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) ;
where the R⁷, R⁸ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) ,
unbranched or branched acyl group which has 1 to 30 carbon atoms and in which at least one hydrogen radical may be replaced by a radical selected from the group consisting of -OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) ;
and where, when p⁹ = 1, -NR³R⁴ may also be a radical of the chemical structure **(x),**
and where, when p¹⁰ = 1, -NR¹¹R¹² may also be a radical of the chemical structure **(x),**
and where the -NR⁵R⁶, -NR⁷R⁸, -NR⁹R¹⁰ radicals may each independently also be a radical of the chemical structure **(X)** ,
where the chemical structure **(x)** is defined as follows: 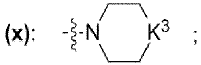
where K³ is selected from the group consisting of -O-, - S-, -NH-, -N(CH₃)-, -N ( CH₂CH₃ ) -;
where the R²¹, R²², R²³, R²⁴, R²⁵, R²⁶ radicals are each independently selected from the group consisting of
hydrogen,
unbranched or branched alkyl group having 1 to 30 carbon atoms,
a group having the chemical structure **(xi)** with 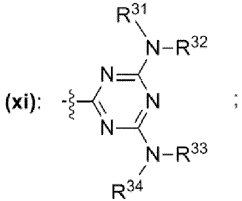 where the R²⁷, R²⁸, R²⁹, R³¹, R³², R³³, R³⁴ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) ,
unbranched or branched acyl group which has 1 to 30 carbon atoms and in which at least one hydrogen radical may be replaced by a radical selected from the group consisting of -OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) ;
a radical having a chemical structure selected from the group consisting of **(xii), (xiii), (xiv), (xv), (xvi), (xvii), (xviii)** with 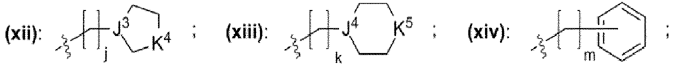 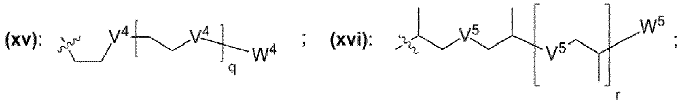 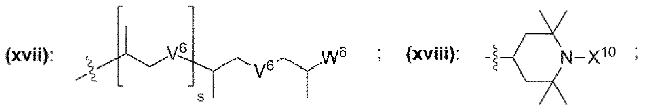
where J³, J⁴ are each independently selected from the group consisting of CH, N,
where K⁴, K⁵ are each independently selected from the group consisting of -O-, -NH-, -N(CH₃)-, -N(CH₂CH₃)-, -S-, -CH₂-, where V⁴, V⁵, V⁶ are each independently selected from the group consisting of -O-, -S-, -NH-, - NR" '- with R' " = unbranched or branched alkyl group having 1 to 6 carbon atoms,
where W⁴, W⁵, W⁶ are each independently selected from the group consisting of H, methyl, ethyl,
where j, k, m, q, r, s are each independently an integer from the range of 0 to 50,
where X¹⁰ is selected from the group consisting of hydrogen, -OH, -O., unbranched or branched alkyl group having 1 to 10 carbon atoms, unbranched or branched alkoxy group having 1 to 10 carbon atoms,
where, in the chemical structures **(xii), (xiii), (xiv), (xv), (xvi), (xvii), (xviii),** at least one hydrogen radical bonded to a carbon atom may be replaced by a radical selected from the group consisting of -OH, - NH₂, -OCH₃, -OCH₂CH₃, -NH (CH₃) , -N (CH₃) ₂, -NH (CH₂CH₃) , -N (CH₂CH₃) ₂, -N (CH₃) (CH₂CH₃) ,
and with the proviso that R²¹ and R²⁶, when p¹⁷ = p¹⁸ = p¹⁹ = p²⁰ = 0, may each independently also be a group of the chemical structure **(xix)** with 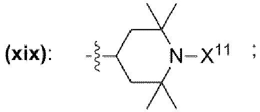 where X¹¹ is selected from the group consisting of hydrogen, OH, -O., unbranched or branched alkyl group having 1 to 10 carbon atoms, unbranched or branched alkoxy group having 1 to 10 carbon atoms,
and wherein reductive conditions are established by reacting the at least one triacetonamine compound **(I)** with formaldehyde in the presence of hydrogen and in the presence of an unsupported catalyst, where the unsupported catalyst includes at least one metal **M,** where the metal **M** is selected from the group consisting of Ag, V, Cr, Mo, Mn, Ni, Pd, Pt, Fe, Ru, Os, Co, Rh, Ir, Cu.

2. Process according to Claim 1, wherein p¹ = p² = p³ = p⁴ = p⁵ = p⁶ = p⁷ = p⁸ = p¹¹ = p¹² = p¹³ = p¹⁴ = 0 and where p⁹, p¹⁰, p¹⁵, p¹⁶, p¹⁷, p¹⁸, p^{19,} p²⁰ are each independently 0 or 1.

3. Process according to Claim 1 or 2, wherein
Y¹, Y², Y³, Y⁴, Y⁵, Y⁶, Y⁷, Y⁸, Y⁹, Y¹⁰ are each independently selected from the group consisting of
unbranched or branched alkylene group having 1 to 30 carbon atoms,
divalent saturated hydrocarbyl group having 3 to 30 carbon atoms and having at least one saturated ring composed of 3 to 30 carbon atoms.

4. Process according to one or more of Claims 1 to 3, wherein the R¹, R^{2,} R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N (CH₃) ₂, - NH (CH₂CH₃), -N (CH₂CH₃) ₂, -N (CH₃) (CH₂CH₃) ,
a radical having a chemical structure **(ix)** with 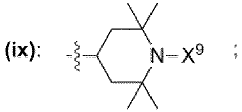 where X⁹ is selected from the group consisting of hydrogen, -OH, -O., unbranched or branched alkyl group having 1 to 10 carbon atoms, unbranched or branched alkoxy group having 1 to 10 carbon atoms;
where the R⁷, R⁸ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃) ₂, - NH (CH₂CH₃), -N (CH₂CH₃) ₂, -N (CH₃) (CH₂CH₃) ,
and where, when p⁹ = 1, -NR³R⁴ may also be a radical of the chemical structure **(x),**
and where, when p¹⁰ = 1, -NR¹¹R¹² may also be a radical of the chemical structure **(x),**
and where the -NR⁵R⁶, -NR⁷R⁸, -NR⁹R¹⁰ radicals may each independently also be a radical of the chemical structure **(x)**,
where the chemical structure **(x)** is defined as follows: (x): 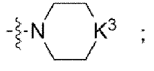 where K³ is selected from the group consisting of -O-, - S-, -NH-, -N(CH₃)-, -N (CH₂CH₃) -;
where the R²¹, R²², R²³, R²⁴, R²⁵, R²⁶ radicals are each independently selected from the group consisting of
hydrogen,
unbranched or branched alkyl group having 1 to 30 carbon atoms,
a group having the chemical structure **(xi)** with 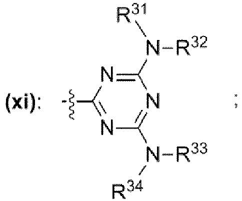 where the R²⁷, R²⁸, R²⁹, R³¹, R³², R³³, R³⁴ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, - NH(CH₂CH₃), -N(CH₂CH₃)₂, -N(CH₃)(CH₂CH₃),
a radical having a chemical structure **(xviii)** with 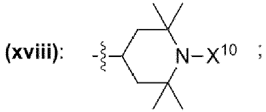 where X¹⁰ is selected from the group consisting of hydrogen, -OH, -O·, unbranched or branched alkyl group having 1 to 10 carbon atoms, unbranched or branched alkoxy group having 1 to 10 carbon atoms,
and with the proviso that R²¹ and R²⁶, when p¹⁷ = p¹⁸ = p¹⁹ = p²⁰ = 0, may each independently also be a group of the chemical structure **(xix)** with 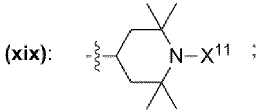 where X¹¹ is selected from the group consisting of hydrogen, OH, -O·, unbranched or branched alkyl group having 1 to 10 carbon atoms, unbranched or branched alkoxy group having 1 to 10 carbon atoms.

5. Process according to one or more of Claims 1 to 4, wherein
X⁴ = X⁵ = X⁶ = X⁷ = X⁸ = X⁹ = X¹⁰ = X¹¹ = hydrogen.

6. Process according to one or more of Claims 1 to 5, wherein the triacetonamine compound **(I)** is selected from the group consisting of the chemical structures **(I-A), (I-B), (I-C), (I-D), (I-E).**

7. Process according to one or more of Claims 1 to 6, wherein the triacetonamine compound **(I)** is selected from the group consisting of the chemical structures **(I-A), (I-B), (I-D),**
and where Z¹ is selected from the group consisting of - O-_{,} -S-, -NR³⁰-;
where the R¹, R², R³⁰ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N (CH₃) ₂, - NH (CH₂CH₃) , -N (CH₂CH₃) ₂, -N (CH₃) (CH₂CH₃) .

8. Process according to one or more of Claims 1 to 7, wherein the triacetonamine compound **(I)** is selected from the group consisting of the chemical structures **(I-A), (I-B), (I-D),**
and where Z¹ is selected from the group consisting of - O-, -NR³⁰-;
where the R¹, R², R³⁰ radicals are each independently selected from the group consisting of
hydrogen,
unbranched or branched alkyl group having 1 to 12 carbon atoms.

9. Process according to one or more of Claims 1 to 8, wherein formaldehyde is used as a gas, as an aqueous solution or as a solid.

10. Process according to one or more of Claims 1 to 9, wherein the at least one triacetonamine compound **(I)** is reacted with formaldehyde under reductive conditions in at least one solvent, where the solvent is selected from the group consisting of aliphatic solvents, aromatic solvents, ethers, halogenated solvents, amides, thio compounds, carboxylic acids, alcohols, water.

11. Process according to one or more of Claims 1 to 10, wherein the at least one triacetonamine compound **(I)** is reacted with formaldehyde under reductive conditions at a temperature in the range from 20°C to 350°C and a pressure in the range from 2 bar to 500 bar.

12. Process according to one or more of Claims 1 to 11, wherein the metal **M** is selected from the group consisting of Ni, Co, Cu, Fe, Ag.

## Revendications

1. Procédé de fabrication d'un composé de triacétonamine à substitution N-méthyle,
**caractérisé en ce qu'**au moins un composé de triacétonamine **(I)** est mis en réaction avec du formaldéhyde dans des conditions réductrices,
le composé de triacétonamine **(I)** étant choisi dans le groupe constitué par les structures chimiques **(I-A), (I-B), (I-C), (I-D), (I-E), (I-F), (I-G), (I-H)** avec 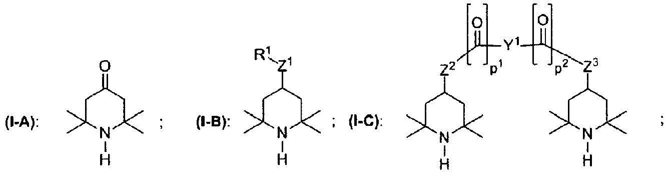 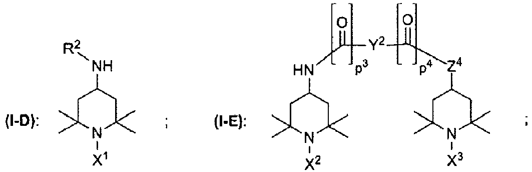 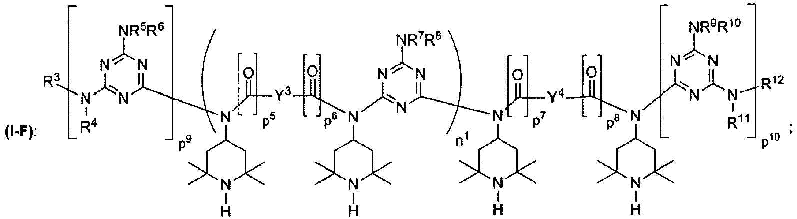 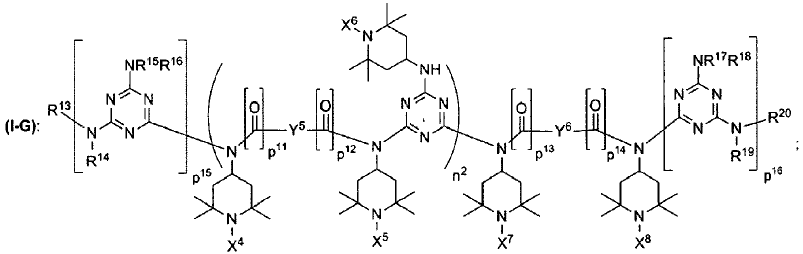 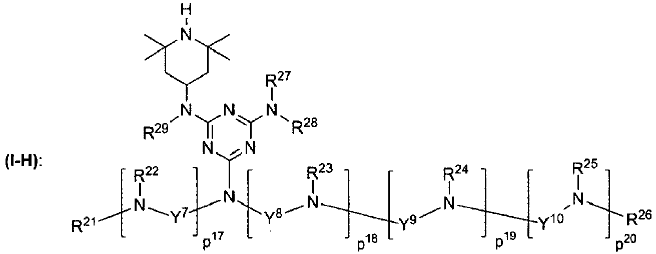
dans lesquelles n¹, n² sont chacun indépendamment l'un de l'autre un nombre entier dans la plage allant de 1 à 20 ;
dans lesquelles p¹, p², p³, p⁴, p⁵, p⁶, p⁷, p⁸, p⁹, p¹⁰, p¹¹, p¹², p¹³, p¹⁴, p¹⁵, p¹⁶, p¹⁷, p¹⁸, p^{19,} p²⁰ sont chacun indépendamment les uns des autres 0 ou 1 ;
dans lesquelles X¹ est choisi dans le groupe constitué par OH, -O·, un groupe alkyle non ramifié ou ramifié contenant 1 à 10 atomes de carbone, un groupe alcoxy non ramifié ou ramifié contenant 1 à 10 atomes de carbone ;
dans lesquelles X², X³ sont choisis dans le groupe constitué par l'hydrogène, OH, -O·, un groupe alkyle non ramifié ou ramifié contenant 1 à 10 atomes de carbone, un groupe alcoxy non ramifié ou ramifié contenant 1 à 10 atomes de carbone, et dans lesquelles X², X³ sont chacun choisis indépendamment l'un de l'autre, à l'exception que l'équation suivante n'est pas satisfaite : X² = X³ = hydrogène ;
dans lesquelles X⁴, X⁵, X⁶, X⁷, X⁸ sont chacun choisis indépendamment les uns des autres dans le groupe constitué par l'hydrogène, OH, -O·, un groupe alkyle non ramifié ou ramifié contenant 1 à 10 atomes de carbone, un groupe alcoxy non ramifié ou ramifié contenant 1 à 10 atomes de carbone ;
dans lesquelles Y¹, Y², Y³, Y⁴, Y⁵, Y⁶, Y⁷, Y⁸, Y⁹, Y¹⁰ sont chacun choisis indépendamment les uns des autres dans le groupe constitué par :
un groupe alkylène non ramifié ou ramifié contenant 1 à 30 atomes de carbone,
un groupe hydrocarboné saturé bivalent contenant 3 à 30 atomes de carbone, qui comprend au moins un cycle saturé constitué par 3 à 30 atomes de carbone,
un groupe hydrocarboné bivalent contenant 6 à 30 atomes de carbone, parmi lesquels au moins 6 atomes de carbone se trouvent dans un système aromatique et les autres atomes de carbone, s'ils sont présents, sont saturés,
un radical pontant, qui présente une structure chimique choisie dans le groupe constitué par (i), (ii), avec 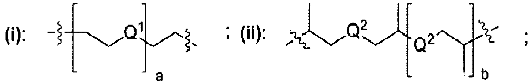 dans lesquelles
Q¹, Q² sont chacun choisis indépendamment l'un de l'autre dans le groupe constitué par -O-, -S-, -NH- ou -NR'- avec R' = un groupe alkyle non ramifié ou ramifié contenant 1 à 6 atomes de carbone,
dans lesquelles a est un nombre entier choisi dans la plage allant de 1 à 50,
dans lesquelles b est un nombre entier choisi dans la plage allant de 0 à 50,
et dans lesquelles Y¹ peut également être une liaison directe, si au moins un indice parmi p¹ et p² présente la valeur 1,
et dans lesquelles Y² peut également être une liaison directe, si au moins un indice parmi p³ et p⁴ présente la valeur 1,
et dans lesquelles Y³ peut également être une liaison directe, si au moins un indice parmi p⁵ et p⁶ présente la valeur 1,
et dans lesquelles Y⁴ peut également être une liaison directe, si au moins un indice parmi p⁷ et p⁸ présente la valeur 1,
et dans lesquelles Y⁵ peut également être une liaison directe, si au moins un indice parmi p¹¹ et p¹² présente la valeur 1,
et dans lesquelles Y⁶ peut également être une liaison directe, si au moins un indice parmi p¹³ et p¹⁴ présente la valeur 1,
et dans lesquelles Z¹, Z², Z³, Z⁴ sont chacun choisis indépendamment les uns des autres dans le groupe constitué par -O-, -S-, -NR³⁰- ;
dans lesquelles les radicaux R¹, R^{2,} R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ sont chacun choisis indépendamment les uns des autres dans le groupe constitué par :
l'hydrogène,
un groupe alkyle non ramifié ou ramifié contenant 1 à 30 atomes de carbone, dans lequel au moins un radical hydrogène peut être remplacé par un radical choisi dans le groupe constitué par -OH, -NH₂, -OCH₃, - OCH₂CH₃, -NH(CH₃), -N(CH₃)₂, -NH(CH₂CH₃), -N(CH₂CH₃)₂, - N (CH₃) (CH₂CH₃) ,
un groupe acyle non ramifié ou ramifié contenant 1 à 30 atomes de carbone, dans lequel au moins un radical hydrogène peut être remplacé par un radical choisi dans le groupe constitué par -OH, -NH₂, -OCH₃, -OCH₂CH₃, - NH (CH₃) , -N (CH₃) ₂, -NH (CH₂CH₃) , -N (CH₂CH₃) ₂, - N (CH₃) (CH₂CH₃) ,
un radical, qui présente une structure chimique choisie dans le groupe constitué par **(iii), (iv), (v), (vi), (vii), (viii), (ix),** avec : 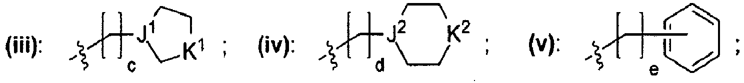 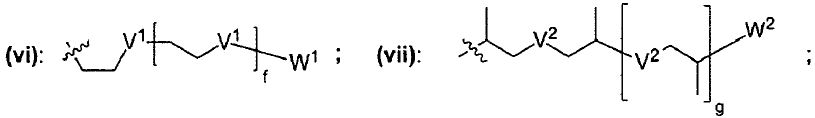 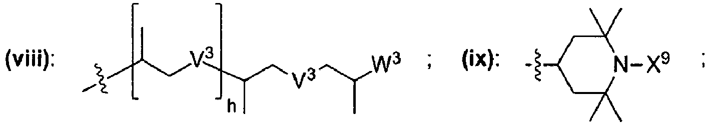
dans lesquelles J¹, J² sont chacun choisis indépendamment l'un de l'autre dans le groupe constitué par CH, N,
dans lesquelles K¹, K² sont chacun choisis indépendamment l'un de l'autre dans le groupe constitué par -O-, -NH-, -N(CH₃)-, -N(CH₂CH₃) -, -S-, -CH₂-,
dans lesquelles V¹, V², V³ sont chacun choisis indépendamment les uns des autres dans le groupe constitué par -O-, -S-, -NH-, -NR"- avec R" = un groupe alkyle non ramifié ou ramifié contenant 1 à 6 atomes de carbone,
dans lesquelles W¹, W², W³ sont chacun choisis indépendamment les uns des autres dans le groupe constitué par H, méthyle, éthyle,
dans lesquelles c, d, e, f, g, h sont chacun indépendamment les uns des autres un nombre entier de la plage allant de 0 à 50,
dans lesquelles X⁹ est choisi dans le groupe constitué par l'hydrogène, -OH, -O·, un groupe alkyle non ramifié ou ramifié contenant 1 à 10 atomes de carbone, un groupe alcoxy non ramifié ou ramifié contenant 1 à 10 atomes de carbone,
dans les structures chimiques **(iii), (iv), (v), (vi), (vii), (viii), (ix),** au moins un radical hydrogène relié à un atome de carbone pouvant être remplacé par un radical choisi dans le groupe constitué par :
-OH, -NH₂, -OCH₃, -OCH₂CH₃, -NH(CH₃), -N(CH₃)₂, - NH(CH₂CH₃), -N(CH₂CH₃)₂, -N(CH₃)(CH₂CH₃) ;
dans lesquelles les radicaux R⁷, R⁸ sont chacun choisis indépendamment les uns des autres dans le groupe constitué par :
l'hydrogène,
un groupe alkyle non ramifié ou ramifié contenant 1 à 30 atomes de carbone, dans lequel au moins un radical hydrogène peut être remplacé par un radical choisi dans le groupe constitué par -OH, -NH₂, -OCH₃, - OCH₂CH₃, -NH(CH₃), -N(CH₃)₂, -NH(CH₂CH₃), -N(CH₂CH₃)₂, - N (CH₃) (CH₂CH₃) ,
un groupe acyle non ramifié ou ramifié contenant 1 à 30 atomes de carbone, dans lequel au moins un radical hydrogène peut être remplacé par un radical choisi dans le groupe constitué par -OH, -NH₂, -OCH₃, -OCH₂CH₃, - NH(CH₃), -N(CH₃)₂, -NH(CH₂CH₃), -N(CH₂CH₃)₂, - N(CH₃)(CH₂CH₃),
et dans lesquelles, lorsque p⁹ = 1, -NR³R⁴ peut également être un radical de la structure chimique (x), et dans lesquelles, lorsque p¹⁰ = 1, -NR¹¹R¹² peut également être un radical de la structure chimique (x), et dans lesquelles les radicaux -NR⁵R⁶, -NR⁷R⁸, -NR⁹R¹⁰ peuvent chacun indépendamment les uns des autres également être un radical de la structure chimique **(x),** dans lesquelles la structure chimique **(x)** est définie par : 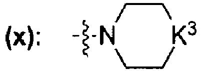 dans laquelle K³ est choisi dans le groupe constitué par -O-, -S-, -NH-, -N(CH₃)-, -N(CH₂CH₃)- ;
dans laquelle les radicaux R²¹, R²², R²³, R²⁴, R²⁵, R²⁶ sont chacun choisis indépendamment les uns des autres dans le groupe constitué par :
l'hydrogène,
un groupe alkyle non ramifié ou ramifié contenant 1 à 30 atomes de carbone,
un groupe ayant la structure chimique **(xi),** avec 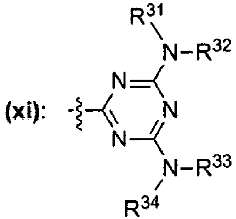 dans laquelle les radicaux R²⁷, R²⁸, R²⁹, R³¹, R³², R³³, R³⁴ sont chacun choisis indépendamment les uns des autres dans le groupe constitué par :
l'hydrogène,
un groupe alkyle non ramifié ou ramifié contenant 1 à 30 atomes de carbone, dans lequel au moins un radical hydrogène peut être remplacé par un radical choisi dans le groupe constitué par -OH, -NH₂, -OCH₃, - OCH₂CH₃, -NH(CH₃), -N(CH₃)₂, -NH(CH₂CH₃), -N(CH₂CH₃)₂, - N (CH₃) (CH₂CH₃) ,
un groupe acyle non ramifié ou ramifié contenant 1 à 30 atomes de carbone, dans lequel au moins un radical hydrogène peut être remplacé par un radical choisi dans le groupe constitué par -OH, -NH₂, -OCH₃, -OCH₂CH₃,-NH(CH₃) , -N(CH₃)₂, -NH(CH₂CH₃), -N(CH₂CH₃)₂,-N(CH₃)(CH₂CH₃),
un radical qui présente une structure chimique choisie dans le groupe constitué par **(xii), (xiii), (xiv), (xv), (xvi), (xvii), (xviii),** avec : 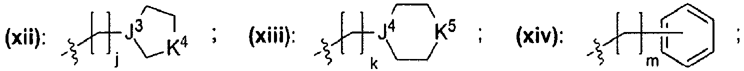 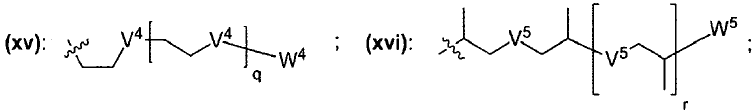 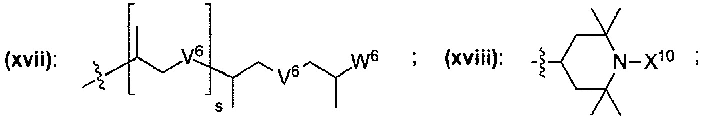
dans lesquelles J³, J⁴ sont chacun choisis indépendamment l'un de l'autre dans le groupe constitué par CH, N,
dans lesquelles K⁴, K⁵ sont chacun choisis indépendamment l'un de l'autre dans le groupe constitué par -O-, -NH-, -N(CH₃)-, -N(CH₂CH₃) -, -S-, -CH₂-,
dans lesquelles V⁴, V⁵, V⁶ sont chacun choisis indépendamment les uns des autres dans le groupe constitué par -O-, -S-, -NH-, -NR''' - avec R''' = un groupe alkyle non ramifié ou ramifié contenant 1 à 6 atomes de carbone,
dans lesquelles W⁴, W⁵, W⁶ sont chacun choisis indépendamment les uns des autres dans le groupe constitué par H, méthyle, éthyle,
dans lesquelles j, k, m, q, r, s sont chacun indépendamment les uns des autres un nombre entier dans la plage allant de 0 à 50,
dans lesquelles X¹⁰ est choisi dans le groupe constitué par l'hydrogène, -OH, -O·, un groupe alkyle non ramifié ou ramifié contenant 1 à 10 atomes de carbone, un groupe alcoxy non ramifié ou ramifié contenant 1 à 10 atomes de carbone,
dans les structures chimiques **(xii), (xiii), (xiv), (xv), (xvi), (xvii), (xviii),** au moins un radical hydrogène relié à un atome de carbone pouvant être remplacé par un radical choisi dans le groupe constitué par -OH, -NH₂, -OCH₃, -OCH₂CH₃, -NH (CH₃) , -N(CH₃) ₂, - NH(CH₂CH₃), -N(CH₂CH₃)₂, -N(CH₃)(CH₂CH₃),
et à condition que R²¹ et R²⁶ pour p¹⁷ = p¹⁸ = p¹⁹ = p²⁰ = 0 puissent chacun indépendamment les uns des autres également être un groupe de la structure chimique **(xix)** : 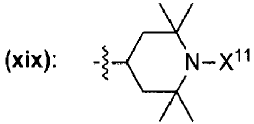 dans laquelle X¹¹ est choisi dans le groupe constitué par hydrogène, OH, -O·,un groupe alkyle non ramifié ou ramifié contenant 1 à 10 atomes de carbone, un groupe alcoxy non ramifié ou ramifié contenant 1 à 10 atomes de carbone,
et des conditions réductrices étant ajustées par mise en réaction dudit au moins un composé de triacétonamine **(I)** avec du formaldéhyde en présence d'hydrogène et en présence d'un catalyseur plein, le catalyseur plein comprenant au moins un métal **M,** le métal **M** étant choisi dans le groupe constitué par Ag, V, Cr, Mo, Mn, Ni, Pd, Pt, Fe, Ru, Os, Co, Rh, Ir, Cu.

2. Procédé selon la revendication 1, dans lequel p¹ = p² = p³ = p⁴ = p⁵ = p⁶ = p⁷ = p⁸ = p¹¹ = p¹² = p¹³= p¹⁴ = 0 et dans lequel p⁹, p¹⁰, p¹⁵, p¹⁶, p¹⁷, p¹⁸, p^{19,} p²⁰ sont chacun indépendamment les uns des autres 0 ou 1.

3. Procédé selon la revendication 1 ou 2, dans lequel Y¹, Y², Y³, Y⁴, Y⁵, Y⁶, Y⁷, Y⁸, Y⁹, Y¹⁰ sont chacun choisis indépendamment les uns des autres dans le groupe constitué par :
un groupe alkylène non ramifié ou ramifié contenant 1 à 30 atomes de carbone,
un groupe hydrocarboné saturé bivalent contenant 3 à 30 atomes de carbone, qui comprend au moins un cycle saturé constitué par 3 à 30 atomes de carbone.

4. Procédé selon une ou plusieurs des revendications 1 à 3, dans lequel les radicaux R¹, R², R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ sont chacun choisis indépendamment les uns des autres dans le groupe constitué par :
l'hydrogène,
un groupe alkyle non ramifié ou ramifié contenant 1 à 30 atomes de carbone, dans lequel au moins un radical hydrogène peut être remplacé par un radical choisi dans le groupe constitué par -OH, -NH₂, -OCH₃, - OCH₂CH₃, -NH(CH₃), -N(CH₃)₂, -NH(CH₂CH₃), -N(CH₂CH₃)₂, - N(CH₃)(CH₂CH₃),
un radical qui présente une structure chimique **(ix)** avec 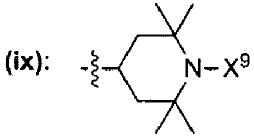 dans laquelle X⁹ est choisi dans le groupe constitué par l'hydrogène, -OH, -O·, un groupe alkyle non ramifié ou ramifié contenant 1 à 10 atomes de carbone, un groupe alcoxy non ramifié ou ramifié contenant 1 à 10 atomes de carbone ;
dans lequel les radicaux R⁷, R⁸ sont chacun choisis indépendamment les uns des autres dans le groupe constitué par :
l'hydrogène,
un groupe alkyle non ramifié ou ramifié contenant 1 à 30 atomes de carbone, dans lequel au moins un hydrogène peut être remplacé par un radical choisi dans le groupe constitué par -OH, -NH₂, -OCH₃, -OCH₂CH₃, - NH(CH₃), -N(CH₃)₂, -NH(CH₂CH₃), -N(CH₂CH₃)₂, - N(CH₃)(CH₂CH₃),
et dans lequel, lorsque p⁹ = 1, -NR³R⁴ peut également être un radical de la structure chimique **(x),**
et dans lequel, lorsque p¹⁰ = 1, -NR¹¹R¹² peut également être un radical de la structure chimique **(x),**
et dans lequel les radicaux -NR⁵R⁶, -NR⁷R⁸, -NR⁹R¹⁰ peuvent chacun indépendamment les uns des autres également être un radical de la structure chimique **(x),** dans lequel la structure chimique **(x)** est définie par : 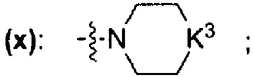 dans laquelle K³ est choisi dans le groupe constitué par -O-, -S-, -NH-, -N(CH₃)-, -N(CH₂CH₃)- ;
dans lequel les radicaux R²¹, R²², R²³, R²⁴, R²⁵, R²⁶ sont chacun choisis indépendamment les uns des autres dans le groupe constitué par :
l'hydrogène,
un groupe alkyle non ramifié ou ramifié contenant 1 à 30 atomes de carbone,
un groupe présentant la structure chimique **(xi)** avec 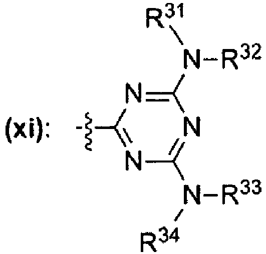 dans laquelle les radicaux R²⁷, R²⁸, R²⁹, R³¹, R³², R³³, R³⁴ sont chacun indépendamment les uns des autres choisis dans le groupe constitué par :
l'hydrogène,
un groupe alkyle non ramifié ou ramifié contenant 1 à 30 atomes de carbone, dans lequel au moins un radical hydrogène peut être remplacé par un radical choisi dans le groupe constitué par -OH, -NH₂, -OCH₃,-OCH₂CH₃, -NH(CH₃), -N(CH₃)₂, -NH(CH₂CH₃), -N(CH₂CH₃)₂,-N (CH₃) (CH₂CH₃),
un radical, qui présente une structure chimique **(xviii)** avec 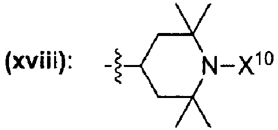 dans laquelle X¹⁰ est choisi dans le groupe constitué par l'hydrogène, -OH, -O·, un groupe alkyle non ramifié ou ramifié contenant 1 à 10 atomes de carbone, un groupe alcoxy non ramifié ou ramifié contenant 1 à 10 atomes de carbone,
et à condition que R²¹ et R²⁶ pour p¹⁷ = p¹⁸ = p¹⁹ = p²⁰ = 0 puissent chacun indépendamment l'un de l'autre également être un groupe de la structure chimique **(xix)** avec 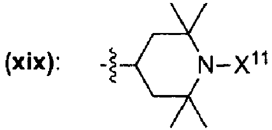 dans laquelle X¹¹ est choisi dans le groupe constitué par l'hydrogène, OH, -O·, un groupe alkyle non ramifié ou ramifié contenant 1 à 10 atomes de carbone, un groupe alcoxy non ramifié ou ramifié contenant 1 à 10 atomes de carbone.

5. Procédé selon une ou plusieurs des revendications 1 à 4, dans lequel
X⁴ = X⁵ = X⁶ = X⁷ = X⁸ = X⁹ = X¹⁰ = X¹¹ = hydrogène.

6. Procédé selon une ou plusieurs des revendications 1 à 5, dans lequel le composé de triacétonamine **(I)** est choisi dans le groupe constitué par les structures chimiques **(I-A), (I-B), (I-C), (I-D), (I-E).**

7. Procédé selon une ou plusieurs des revendications 1 à 6, dans lequel le composé de triacétonamine **(I)** est choisi dans le groupe constitué par les structures chimiques **(I-A), (I-B), (I-D),**
et dans lequel Z¹ est choisi dans le groupe constitué par -O-, -S-, -NR³⁰- ;
dans lequel les radicaux R¹, R^{2,} R³⁰ sont chacun choisis indépendamment les uns des autres dans le groupe constitué par :
l'hydrogène,
un groupe alkyle non ramifié ou ramifié contenant 1 à 30 atomes de carbone, dans lequel au moins un radical hydrogène peut être remplacé par un radical choisi dans le groupe constitué par -OH, -NH₂, -OCH₃, - OCH₂CH₃, -NH(CH₃), -N(CH₃)₂, -NH(CH₂CH₃), -N(CH₂CH₃)₂, - N (CH₃) (CH₂CH₃) .

8. Procédé selon une ou plusieurs des revendications 1 à 7, dans lequel le composé de triacétonamine **(I)** est choisi dans le groupe constitué par les structures chimiques **(I-A), (I-B), (I-D),**
et dans lequel Z¹ est choisi dans le groupe constitué par -O-, -NR³⁰- ;
dans lequel les radicaux R¹, R², R³⁰ sont chacun choisis indépendamment les uns des autres dans le groupe constitué par :
l'hydrogène,
un groupe alkyle non ramifié ou ramifié contenant 1 à 12 atomes de carbone.

9. Procédé selon une ou plusieurs des revendications 1 à 8, dans lequel le formaldéhyde est utilisé sous la forme d'un gaz, sous la forme d'une solution aqueuse ou sous la forme d'un solide.

10. Procédé selon une ou plusieurs des revendications 1 à 9, dans lequel ledit au moins un composé de triacétonamine **(I)** est mis en réaction avec du formaldéhyde dans des conditions réductrices dans au moins un solvant, le solvant étant choisi dans le groupe constitué par les solvants aliphatiques, les solvants aromatiques, les éthers, les solvants halogénés, les amides, les composés thio, les acides carboxyliques, les alcools, l'eau.

11. Procédé selon une ou plusieurs des revendications 1 à 10, dans lequel ledit au moins un composé de triacétonamine **(I)** est mis en réaction avec du formaldéhyde dans des conditions réductrices à une température dans la plage allant de 20 °C à 350 °C et à une pression dans la plage allant de 2 bar à 500 bar.

12. Procédé selon une ou plusieurs des revendications 1 à 11, dans lequel le métal **M** est choisi dans le groupe constitué par Ni, Co, Cu, Fe, Ag.
